# EUROPEAN PATENT APPLICATION

(11) **EP 3 257 835 A1**
(43) Date of publication of application: **20.12.2017**
(21) Application number: 16749322.0
(22) Date of filing: 12.02.2016
(51) Int. Cl.: C07C 43/235, C07C 41/38, C07C 43/205, C07C 43/275, C08G 8/00, G03F 7/11, G03F 7/20, G03F 7/26

(54) **COMPOUND, RESIN, LITHOGRAPHY UNDERLAYER FILM FORMING MATERIAL, LITHOGRAPHY UNDERLAYER FILM FORMING COMPOSITION, LITHOGRAPHY UNDERLAYER FILM, METHOD FOR FORMING RESIST PATTERN, METHOD FOR FORMING CIRCUIT PATTERN, AND METHOD FOR PURIFYING COMPOUND OR RESIN**

(30) Priority: 12.02.2015 JP 2015025371
(71) Applicant: Mitsubishi Gas Chemical Company, Inc., Tokyo 100-8324 (JP)
(72) Inventor: TOIDA, Takumi, Hiratsuka-shi Kanagawa 254-0016 (JP); ECHIGO, Masatoshi, Tokyo 100-8324 (JP); SATO, Takashi, Hiratsuka-shi Kanagawa 254-0016 (JP); MAKINOSHIMA, Takashi, Hiratsuka-shi Kanagawa 254-0016 (JP)
(74) Representative: Müller-Boré & Partner Patentanwälte PartG mbB
(86) International application number: PCT/JP2016/054159
(87) International publication number: WO 2016/129679

(57) **Abstract**

A compound represented by the following formula (1). 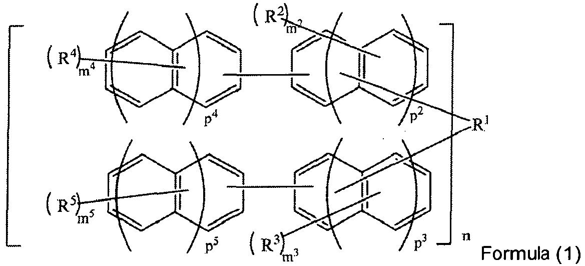 (in formula (1), R¹ represents a 2n-valent group having 1 to 30 carbon atoms, R² to R⁵ each independently represent an alkyl group having 1 to 10 carbon atoms, an aryl group having 6 to 10 carbon atoms, an alkenyl group having 2 to 10 carbon atoms, an alkoxy group having 1 to 30 carbon atoms, a thiol group or a hydroxyl group, provided that at least one R⁴ and/or at least one R⁵ represents an alkoxy group having 1 to 30 carbon atoms, m² and m³ are each independently an integer of 0 to 8, m⁴ and m⁵ are each independently an integer of 0 to 9, provided that m⁴ and m⁵ are not 0 at the same time, n is an integer of 1 to 4, and p² to p⁵ are each independently an integer of 0 to 2.)

## Description

### Technical Field

The present invention relates to a compound or a resin having a specified structure. The present invention also relates to a material for forming an underlayer film for lithography containing the compound or the resin, a composition containing the material, an underlayer film for lithography obtained from the composition, and a resist or circuit pattern forming method using the composition. The present invention further relates to a purification method of the compound or the resin.

### Background Art

Semiconductor devices are manufactured through microfabrication by lithography using a photoresist material, but are required to be made finer by a pattern rule in accordance with the increase in integration degree and the increase in speed of LSI in recent years. In lithography using exposure to light, which is currently used as a general-purpose technique, the resolution is now approaching the intrinsic limitation associated with the wavelength of the light source.

A light source for lithography, for use in forming a resist pattern, has a shorter wavelength from a KrF excimer laser (248 nm) to an ArF excimer laser (193 nm). However, as the resist pattern is made finer and finer, there arise a problem of resolution or a problem of collapse of the resist pattern after development, and therefore there is demanded for making a resist film thinner. However, if the resist film is merely made thinner in response to such a demand, it is difficult to achieve the resist pattern having a film thickness sufficient for processing a substrate. Accordingly, there has been increasingly required a process in which not only the resist pattern but also a resist underlayer film is prepared between a resist and a semiconductor substrate to be processed and the resist underlayer film is allowed to have a function as a mask at the time of processing the substrate.

Currently, as the resist underlayer film for such a process, various ones are known. For example, as one that realizes a resist underlayer film for lithography, having a selection ratio of dry etching rate close to the resist, unlike a conventional resist underlayer film having a high etching rate, there has been proposed a material for forming an underlayer film for multilayer resist process, containing a resin component having at least a substituent which releases a terminal group to form a sulfonic acid residue when a predetermined energy is applied, and a solvent (see Patent Literature 1 described below: Japanese Patent Laid-Open No. 2004-177668). In addition, as one that realizes a resist underlayer film for lithography, having a smaller selection ratio of dry etching rate than the resist, there has been proposed a resist underlayer film material including a polymer having a specified repeating unit (see Patent Literature 2 described below: Japanese Patent Laid-Open No. 2004-271838). Furthermore, as one that realizes a resist underlayer film for lithography, having a smaller selection ratio of dry etching rate than the semiconductor substrate, there has been proposed a resist underlayer film material including a polymer formed by co-polymerizing a repeating unit of acenaphthylene, and a substituted or non-substituted repeating unit having a hydroxy group (see Patent Literature 3 described below: Japanese Patent Laid-Open No. 2005-250434).

On the other hand, as a material for allowing such a resist underlayer film to have a high etching resistance, an amorphous carbon underlayer film is well known, which is formed by CVD using methane gas, ethane gas, acetylene gas, or the like as a raw material. However, there is demanded, in terms of process, a resist underlayer film material that can form a resist underlayer film in a wet process such as a spin coating method or screen printing.

In addition, as a material that is excellent in optical characteristics and etching resistance and that is capable of being dissolved in a solvent and being applied to a wet process, the present inventors have proposed a composition for forming an underlayer film for lithography, which contains a naphthalene formaldehyde polymer including a specified constituent unit, and an organic solvent (see Patent Literature 4: International Publication No. WO 2009/072465 and Patent Literature 5 described below: International Publication No. WO 2011/034062) .

Meanwhile, with respect to a forming method of an intermediate layer for use in forming a resist underlayer film in a three-layer process, for example, known are a forming method of a silicon nitride film (see Patent Literature 6 described below: Japanese Patent Laid-Open No. 2002-334869) and a CVD forming method of a silicon nitride film (see Patent Literature 7 described below: International Publication No. WO 2004/066377). In addition, as an intermediate layer material for a three-layer process, known is a material containing a silsesquioxane-based silicon compound (Patent Literature 8: Japanese Patent Laid-Open No. 2007-226170 and Patent Literature 9 described below: Japanese Patent Laid-Open No. 2007-226204).

### Citation List

### Patent Literature

Patent Literature 1: Japanese Patent Laid-Open No. 2004-177668
Patent Literature 2: Japanese Patent Laid-Open No. 2004-271838
Patent Literature 3: Japanese Patent Laid-Open No. 2005-250434
Patent Literature 4: International Publication No. WO 2009/072465
Patent Literature 5: International Publication No. WO 2011/034062
Patent Literature 6: Japanese Patent Laid-Open No. 2002-334869
Patent Literature 7: International Publication No. WO 2004/066377
Patent Literature 8: Japanese Patent Laid-Open No. 2007-226170
Patent Literature 9: Japanese Patent Laid-Open No. 2007-226204

### Summary of Invention

### Technical Problem

As described above, many materials for forming an underlayer film for lithography have been conventionally proposed, but there has not been proposed any material which not only has such a high solvent solubility as to be able to be applied to a wet process such as a spin coating method or screen printing, but also simultaneously satisfies heat resistance and etching resistance at a high level, and a new material is demanded to be developed.

The present invention has been made in view of the above problem, and an object thereof is to provide a compound or a resin having a specified structure, as well as a material for forming an underlayer film for lithography, which can be applied to a wet process and which is useful for forming a photoresist underlayer film excellent in heat resistance and etching resistance, a composition containing the material, an underlayer film for lithography, obtained from the composition, a resist or circuit pattern forming method using the composition, and a purification method of the compound or the resin.

### Solution to Problem

The present inventors have intensively studied to solve the above problem, and as a result, have found that the above problem can be solved by using a compound or a resin having a specified structure, thereby leading to the completion of the present invention. That is, the present invention is as follows.
[1] A compound represented by the following formula (1). 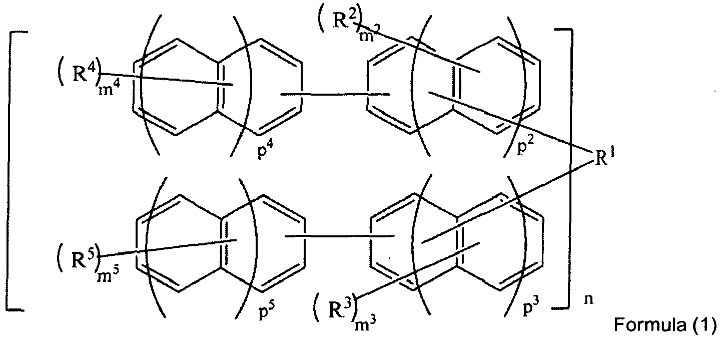 (in formula (1), R¹ represents a 2n-valent group having 1 to 30 carbon atoms, R² to R⁵ each independently represent an alkyl group having 1 to 10 carbon atoms, an aryl group having 6 to 10 carbon atoms, an alkenyl group having 2 to 10 carbon atoms, an alkoxy group having 1 to 30 carbon atoms, a thiol group or a hydroxyl group, provided that at least one R⁴ and/or at least one R⁵ representsrepresents an alkoxy group having 1 to 30 carbon atoms, m² and m³ are each independently an integer of 0 to 8, m⁴ and m⁵ are each independently an integer of 0 to 9, provided that m⁴ and m⁵ are not 0 at the same time, n is an integer of 1 to 4, and p² to p⁵ are each independently an integer of 0 to 2.)
[2] The compound according to the above [1], wherein at least one R² and/or at least one R³ representsrepresents an alkoxy group having 1 to 30 carbon atoms.
[3] The compound according to the above [1] or [2], wherein the compound represented by the formula (1) is a compound represented by the following formula (1a). 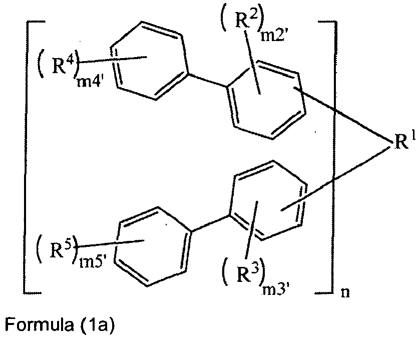 (in formula (1a), R¹ to R⁵ and n are the same as those described in the formula (1), m^{2'} and m^{3'} are each independently an integer of 0 to 4, and m^{4'} and m^{5'} are each independently an integer of 0 to 5, provided that m^{4'} and m^{5'} are not 0 at the same time.)
[4] The compound according to the above [3], wherein the compound represented by the formula (1a) is a compound represented by the following formula (1b). 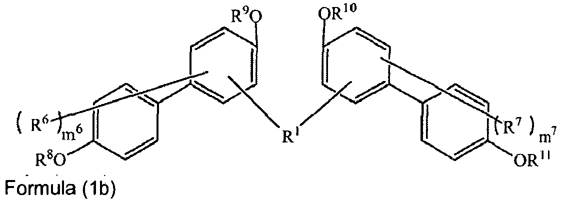 (in formula (1b), R¹ is the same as that described in the formula (1), R⁶ and R⁷ each independently represent an alkyl group having 1 to 10 carbon atoms, an aryl group having 6 to 10 carbon atoms, an alkenyl group having 2 to 10 carbon atoms, a thiol group or a hydroxyl group, R⁸ to R¹¹ each independently represent an alkyl group having 1 to 30 carbon atoms, or a hydrogen atom, provided that at least one of R⁸ to R¹¹ represents an alkyl group having 1 to 30 carbon atoms, and m⁶ and m⁷ are each independently an integer of 0 to 7.)
[5] The compound according to the above [4], wherein the compound represented by the formula (1b) is represented by the following formula (BiF-1-CH). 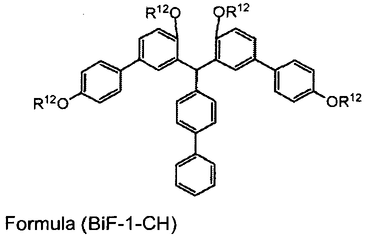 (in formula (BiF-1-CH), each R¹² independently represents a cyclohexyl group or a hydrogen atom, provided that at least one R¹² represents a cyclohexyl group.)
[6] A resin obtained with the compound according to any one of the above [1] to [5] as a monomer.
[7] The resin according to the above [6], which is obtained by a reaction of the compound according to any one of the above [1] to [5] with a compound having crosslinking reactivity.
[8] The resin according to the above [7], wherein the compound having crosslinking reactivity is at least one selected from the group consisting of aldehyde, ketone, carboxylic acid, carboxylic halide, a halogen-containing compound, an amino compound, an imino compound, isocyanate and an unsaturated hydrocarbon group-containing compound.
[9] The resin according to the above [6], having a structure represented by the following formula (2). 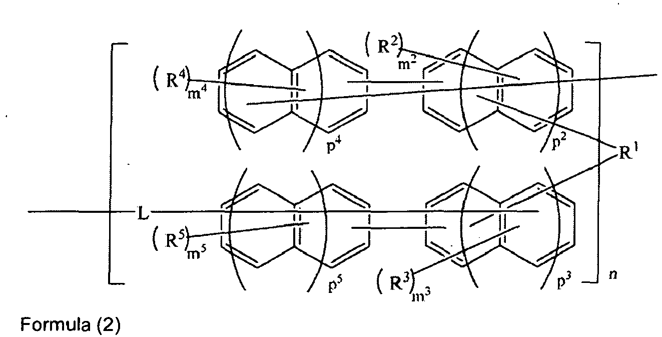 (in formula (2), R¹ represents a 2n-valent group having 1 to 30 carbon atoms, R² to R⁵ each independently represent an alkyl group having 1 to 10 carbon atoms, an aryl group having 6 to 10 carbon atoms, an alkenyl group having 2 to 10 carbon atoms, an alkoxy group having 1 to 30 carbon atoms, a thiol group or a hydroxyl group, provided that at least one R⁴ and/or at least one R⁵ represents an alkoxy group having 1 to 30 carbon atoms, L represents a straight or branched alkylene group having 1 to 20 carbon atoms, or a single bond, m² and m³ are each independently an integer of 0 to 8, m⁴ and m⁵ are each independently an integer of 0 to 9, provided that m⁴ and m⁵ are not 0 at the same time, n is an integer of 1 to 4, and p² to p⁵ are each independently an integer of 0 to 2.)
[10] A material for forming an underlayer film for lithography, comprising the compound according to any one of the above [1] to [5] and/or the resin according to any one of the above [6] to [9].
[11] A composition for forming an underlayer film for lithography, comprising the material for forming an underlayer film for lithography according to the above [10], and a solvent.
[12] The composition for forming an underlayer film for lithography according to the above [11], further comprising a crosslinking agent.
[13] The composition for forming an underlayer film for lithography according to the above [11] or [12], further comprising an acid generator.
[14] An underlayer film for lithography, formed from the composition for forming an underlayer film for lithography according to any of the above [11] to [13].
[15] A resist pattern forming method, comprising forming an underlayer film on a substrate by using the composition for forming an underlayer film according to any of the above [11] to [13], forming at least one photoresist layer on the underlayer film, thereafter irradiating and developing a required region of the photoresist layer with radiation.
[16] A circuit pattern forming method, comprising forming an underlayer film on a substrate by using the composition for forming an underlayer film according to any of the above [11] to [13], forming an intermediate layer film on the underlayer film by using a silicon atom-containing resist intermediate layer film material, forming at least one photoresist layer on the intermediate layer film, thereafter irradiating and developing a required region of the photoresist layer with radiation to form a resist pattern, thereafter etching the intermediate layer film with the resist pattern as a mask, etching the underlayer film with the obtained intermediate layer film pattern as an etching mask, and etching the substrate with the obtained underlayer film pattern as an etching mask, to form a pattern on the substrate.
[17] A purification method of a compound or a resin, comprising a step of bringing a solution including the compound according to any one of the above [1] to [5] or the resin according to any one of the above [6] to [9], and an organic solvent optionally immiscible with water into contact with an acidic aqueous solution for extraction.
[18] The purification method according to the above [17], wherein the acidic aqueous solution is an aqueous solution of at least one mineral acid selected from the group consisting of hydrochloric acid, sulfuric acid, nitric acid and phosphoric acid, or an aqueous solution of at least one organic acid selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid.
[19] The purification method according to the above [17] or [18], wherein the organic solvent optionally immiscible with water is toluene, 2-heptanone, cyclohexanone, cyclopentanone, methyl isobutyl ketone, propylene glycol monomethyl ether acetate or ethyl acetate.
[20] The purification method according to any one of the above [17] to [19], comprising a step of bringing the solution into contact with the acidic aqueous solution to perform an extraction treatment and thereafter further performing an extraction treatment with water.

### Advantageous Effects of Invention

According to the present invention, it is possible to provide a compound or a resin having a specified structure, as well as a material for forming an underlayer film for lithography, which can be applied to a wet process and which is useful for forming a photoresist underlayer film excellent in heat resistance and etching resistance, a composition containing the material, an underlayer film for lithography, obtained from the composition, a resist or circuit pattern forming method using the composition, and a purification method of the compound or the resin.

### Description of Embodiments

Hereinafter, embodiments of the present invention will be described. It is to be noted that the following embodiments are illustrative for describing the present invention, and the present invention is not limited only to such embodiments.

### [Compound and resin of the present invention]

The compound of the present invention is represented by the following formula (1). The compound of the present invention is thus configured to thereby have a high heat resistance, a relatively high carbon concentration, a relatively low oxygen concentration, and also a high solvent solubility.

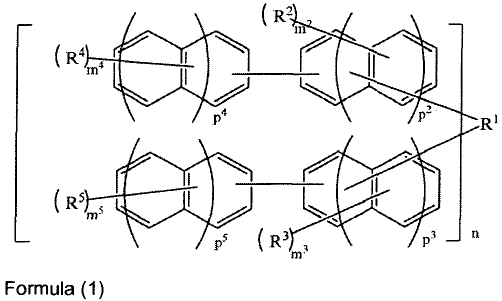

In the formula (1), R¹ represents a 2n-valent group having 1 to 30 carbon atoms, and respective aromatic rings are bonded to each other via R¹. Herein, the 2n-valent group represents an alkylene group having 1 to 30 carbon atoms when n = 1, an alkanetetrayl group having 1 to 30 carbon atoms when n = 2, an alkanehexayl group having 2 to 30 carbon atoms when n = 3, and an alkaneoctayl group having 3 to 30 carbon atoms when n = 4. Examples of the 2n-valent group include those having a straight hydrocarbon group, a branched hydrocarbon group or an alicyclic hydrocarbon group. Herein, the alicyclic hydrocarbon group also includes a bridged alicyclic hydrocarbon group. The 2n-valent group may also have a double bond, a hetero atom, or an aromatic group having 6 to 30 carbon atoms.

In the formula (1), R² to R⁵ each independently represent a monovalent group selected from the group consisting of an alkyl group having 1 to 10 carbon atoms; an aryl group having 6 to 10 carbon atoms; an alkenyl group having 2 to 10 carbon atoms; an alkoxy group having 1 to 30 carbon atoms; a thiol group and a hydroxyl group, provided that at least one R⁴ and/or at least one R⁵ represents an alkoxy group having 1 to 30 carbon atoms.

In the formula (1), m² and m³ are each independently an integer of 0 to 8, and m⁴ and m⁵ are each independently an integer of 0 to 9, but m⁴ and m⁵ are not 0 at the same time.

In the formula (1), n is an integer of 1 to 4.

In the formula (1), p² to p⁵ are each independently an integer of 0 to 2. Herein, for example, when p² is 0, the corresponding aromatic ring is a benzene ring, and when p2 is 1, the corresponding aromatic ring is a naphthalene ring. Furthermore, when p² is 3, the corresponding aromatic ring is a 3-membered aromatic ring such as anthracene.

In the formula (1), the alkyl group having 1 to 10 carbon atoms and the alkenyl group having 2 to 10 carbon atoms each include a straight, branched or cyclic alkyl group or alkenyl group.

In addition, the alkoxy group having 1 to 30 carbon atoms means a group configured from a group selected from a straight hydrocarbon group, a branched hydrocarbon group or an alicyclic hydrocarbon group, an aromatic hydrocarbon group and a group including a combination of two or more thereof, as well as an oxygen atom. Herein, the alicyclic hydrocarbon group also includes a bridged alicyclic hydrocarbon group. The alkoxy group may also have a double bond, a hetero atom, or a halogen atom.

The alkoxy group having 1 to 30 carbon atoms preferably includes a methoxy group, an ethoxy group, a propoxy group, a butoxy group, a pentyloxy group, a hexyloxy group, a cyclobutyloxy group, a cyclopentyloxy group, a cyclohexyloxy group, a cyclohexenyloxy group, an isophoronyloxy group, a norbornanyloxy group, an adamantyloxy group, a tricyclodecanyloxy group, a pyridinyloxy group, a phenyloxy group, a methylphenyloxy group, a dimethylphenyloxy group, an ethylphenyloxy group, a fluorophenyloxy group, a chlorophenyloxy group, a bromophenyloxy group, an iodophenyloxy group, a hydroxyphenyloxy group, a methoxyphenyloxy group, an aminophenyloxy group, a nitrophenyloxy group, a cyanophenyloxy group, a phenylphenyloxy group, a phenyloxyphenyloxy group, a naphthyloxy group, a methylnaphthyloxy group, a dimethylnaphthyloxy group, an ethylnaphthyloxy group, a fluoronaphthyloxy group, a chloronaphthyloxy group, a bromonaphthyloxy group, an iodonaphthyloxy group, a hydroxynaphthyloxy group, a methoxynaphthyloxy group, an aminonaphthyloxy group, a nitronaphthyloxy group, a cyanonaphthyloxy group, a phenylnaphthyloxy group, a phenyloxynaphthyloxy group, an anthracenyloxy group, a pyrenyloxy group and a fluorenyloxy group, more preferably includes a cyclobutyloxy group, a cyclopentyloxy group, a cyclohexyloxy group, a cyclohexenyloxy group, an isophoronyloxy group, a norbornanyloxy group, an adamantyloxy group, a tricyclodecanyloxy group, a pyridinyloxy group, a phenyloxy group, a methylphenyloxy group, a dimethylphenyloxy group, an ethylphenyloxy group, a fluorophenyloxy group, a chlorophenyloxy group, a bromophenyloxy group, an iodophenyloxy group, a hydroxyphenyloxy group, a methoxyphenyloxy group, an aminophenyloxy group, a nitrophenyloxy group, a cyanophenyloxy group, a phenylphenyloxy group, a phenyloxyphenyloxy group, a naphthyloxy group, a methylnaphthyloxy group, a dimethylnaphthyloxy group, an ethylnaphthyloxy group, a fluoronaphthyloxy group, a chloronaphthyloxy group, a bromonaphthyloxy group, an iodonaphthyloxy group, a hydroxynaphthyloxy group, a methoxynaphthyloxy group, an aminonaphthyloxy group, a nitronaphthyloxy group, a cyanonaphthyloxy group, a phenylnaphthyloxy group, a phenyloxynaphthyloxy group, an anthracenyloxy group, a pyrenyloxy group and a fluorenyloxy group, further preferably includes a cyclobutyloxy group, a cyclopentyloxy group, a cyclohexyloxy group, a cyclohexenyloxy group, an isophoronyloxy group, a norbornanyloxy group, an adamantyloxy group, a tricyclodecanyloxy group, a pyridinyloxy group, a phenyloxy group, a methylphenyloxy group, a dimethylphenyloxy group, an ethylphenyloxy group, a methoxyphenyloxy group, a phenylphenyloxy group, a phenyloxyphenyloxy group, a naphthyloxy group, a methylnaphthyloxy group, a dimethylnaphthyloxy group, an ethylnaphthyloxy group, a methoxynaphthyloxy group, a phenylnaphthyloxy group, a phenyloxynaphthyloxy group, an anthracenyloxy group, a pyrenyloxy group and a fluorenyloxy group, and particularly preferably includes a cyclohexyloxy group and a phenyloxy group.

The compound represented by the formula (1) has a high heat resistance due to rigidity of its structure while having a relatively low molecular weight, and therefore it can be used even under a high-temperature baking condition. In addition, the compound has a relatively low molecular weight and a low viscosity, and therefore, even when being applied to a substrate having a step (in particular, fine space, hole pattern and the like), it can be easily filled uniformly in every part of the step. As a result, a material for forming an underlayer film for lithography using such a compound may be improved in terms of embedding properties and planarizing properties in a relatively advantageous manner. In addition, the compound has a relatively high carbon concentration to thereby impart also a high etching resistance. Furthermore, the alkoxy group having 1 to 30 carbon atoms is included to thereby impart further solvent solubility and quality stabilization.

In the compound represented by the formula (1), at least one R² and/or at least one R³ preferably represents an alkoxy group having 1 to 30 carbon atoms in terms of ease of crosslinking and solubility in an organic solvent. In addition, when R² and R³ represent an aryl group having 6 to 10 carbon atoms, having a substituent, the substituent is preferably a substituent including no oxygen atom.

In addition, the compound represented by the formula (1) is preferably a compound represented by the following formula (1a) in terms of raw material supply property.

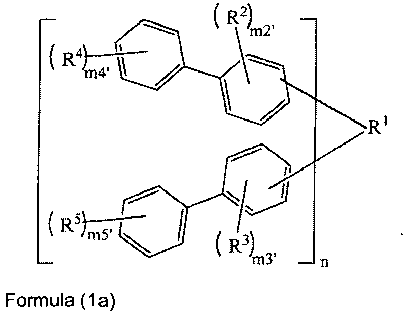

In the formula (1a), R¹ to R⁵ and n are the same as those described in the formula (1).
m^{2'} and m^{3'} are each independently an integer of 0 to 4, and m^{4'} and m^{5'} are each independently an integer of 0 to 5, provided that m^{4'} and m^{5'} are not 0 at the same time.

The compound represented by the formula (1a) is preferably a compound represented by the following formula (1b) in terms of solubility in an organic solvent.

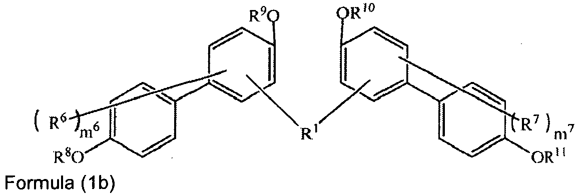

In the formula (1b), R¹ is the same as that described in the formula (1). That is, in the formula (1b), R¹ corresponds to one where n = 1 in the formula (1), and represents a 2-valent group having 1 to 30 carbon atoms as in R¹ in the formula (1).

In the formula (1b), R⁶ and R⁷ each independently represent an alkyl group having 1 to 10 carbon atoms, an aryl group having 6 to 10 carbon atoms, an alkenyl group having 2 to 10 carbon atoms, a thiol group or a hydroxyl group, and R⁸ to R¹¹ each independently represent an alkyl group having 1 to 30 carbon atoms, or a hydrogen atom, provided that at least one of R⁸ to R¹¹ represents an alkyl group having 1 to 30 carbon atoms. The alkyl group and the alkenyl group described above may be each any of straight, branched and cyclic groups.

m⁶ and m⁷ are each independently an integer of 0 to 7.

The compound represented by the formula (1b) is particularly preferably a compound represented by the following formula (BiF-1-CH) in terms of further solubility in an organic solvent.

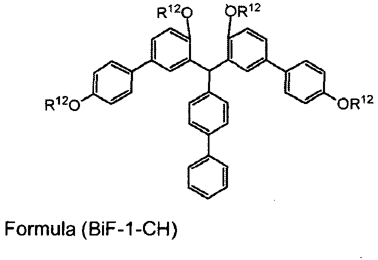

In the formula (BiF-1-CH), each R¹² independently represents a cyclohexyl group or a hydrogen atom in terms of quality stabilization, provided that at least one R¹² represents a cyclohexyl group.

Specific examples of the compound represented by the formula (1) are shown below, but are not limited to those recited herein.

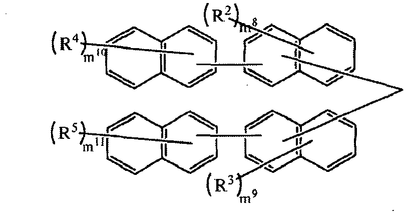

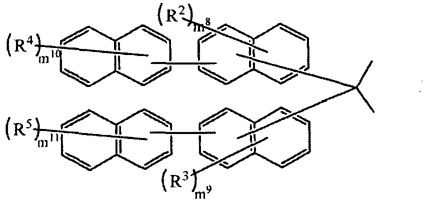

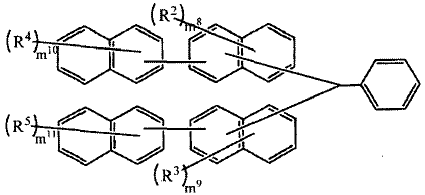

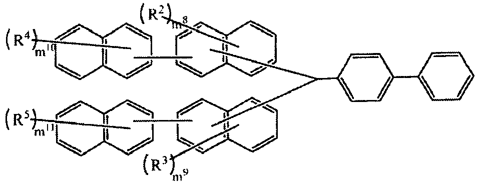

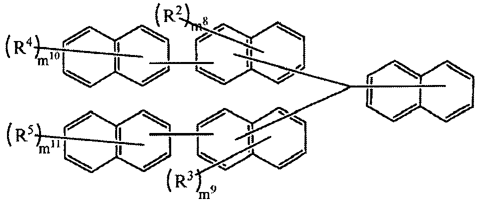

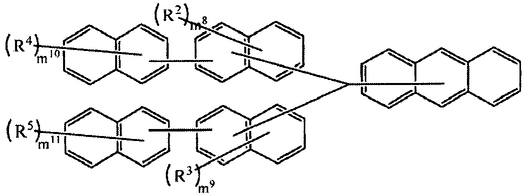

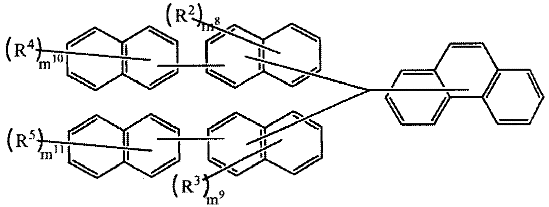

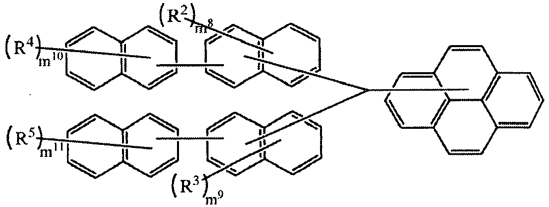

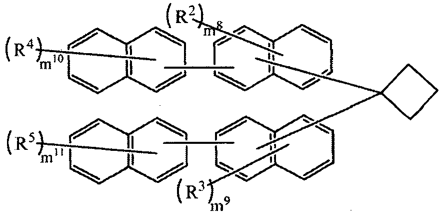

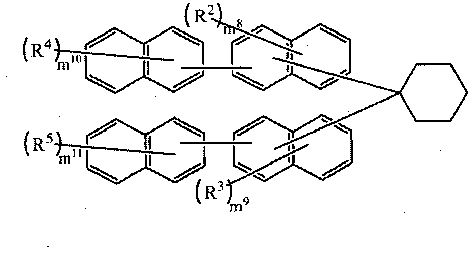

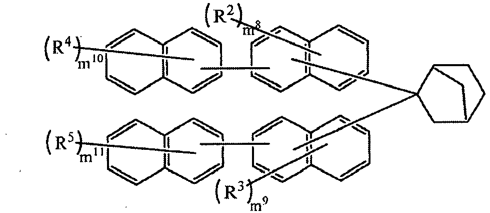

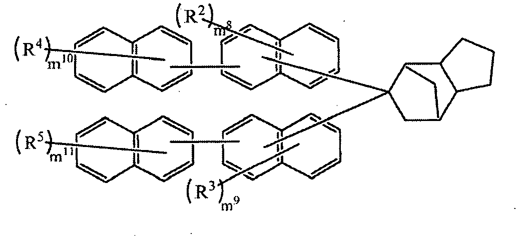

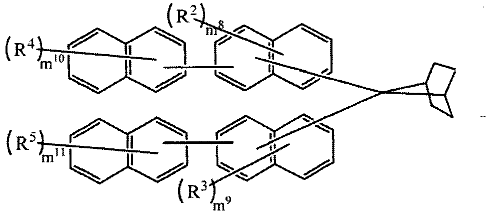

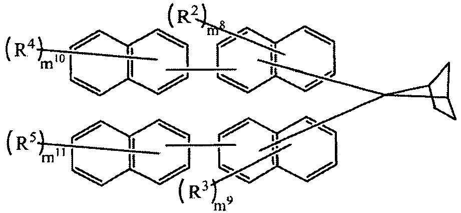

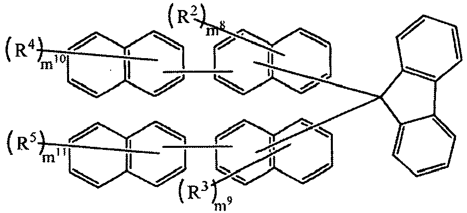

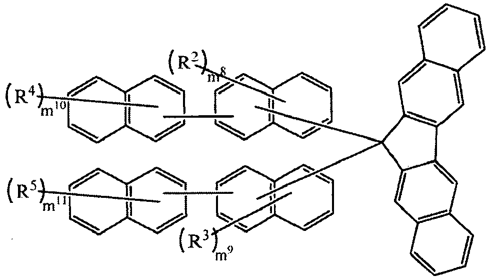

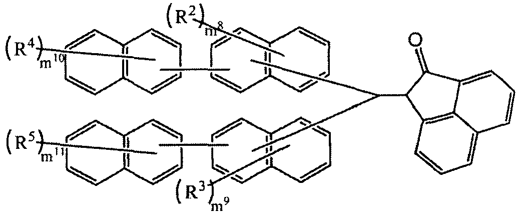

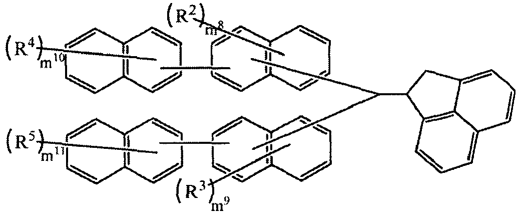

In the above compounds, R² to R⁵ are the same as those described in the formula (1), m⁸ and m⁹ are each independently an integer of 0 to 6, and m¹⁰ and m¹¹ are each independently an integer of 0 to 7, provided that m¹⁰ and m¹¹ are not 0 at the same time.

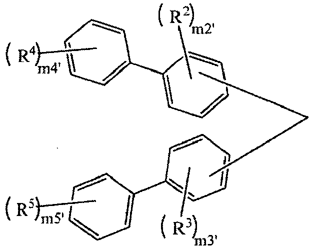

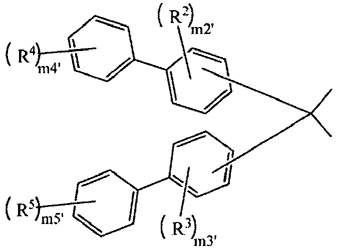

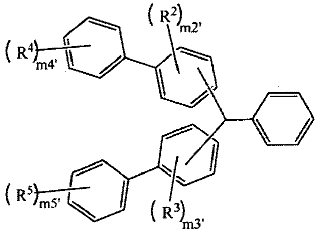

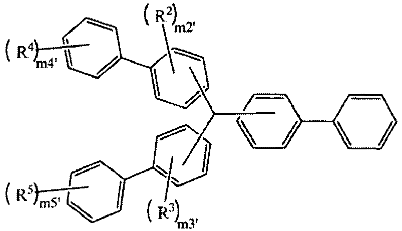

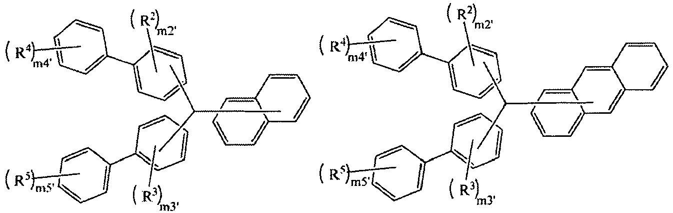

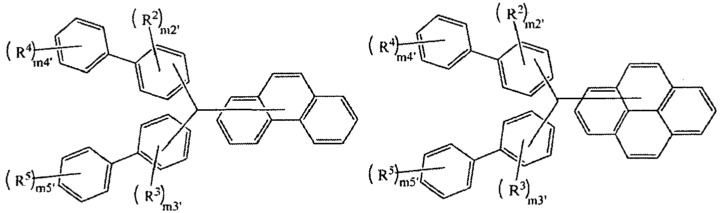

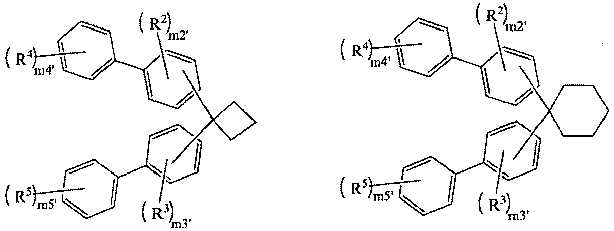

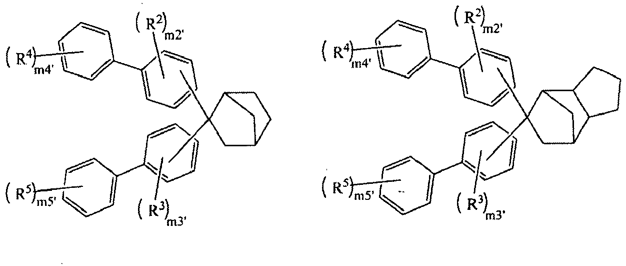

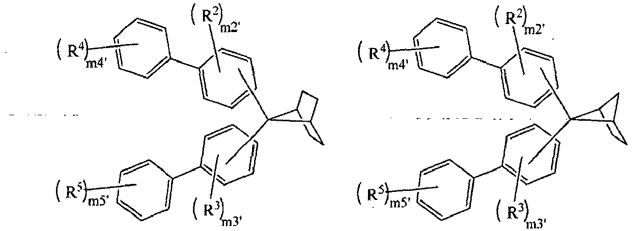

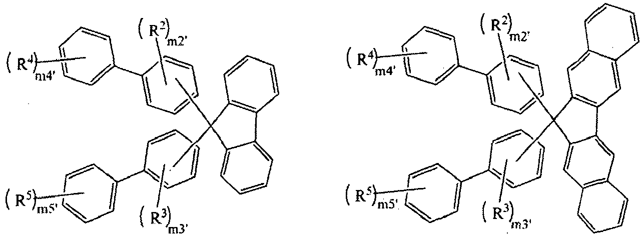

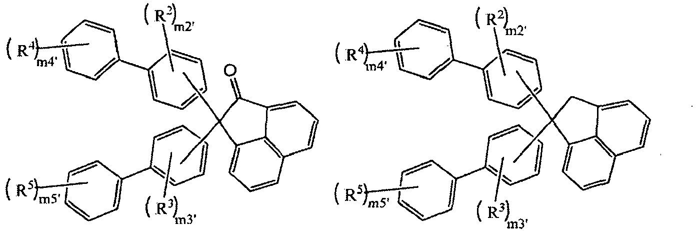

In the compounds, R² to R⁵ are the same as those described in the formula (1). m^{2'} and m^{3'} are each independently an integer of 0 to 4, and m^{4'} and m^{5'} are each independently an integer of 0 to 5, provided that m^{4'} and m^{5'} are not 0 at the same time.

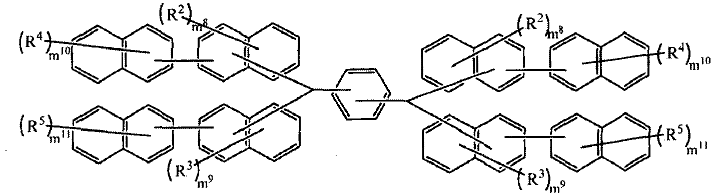

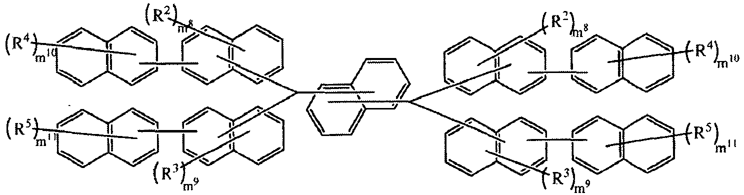

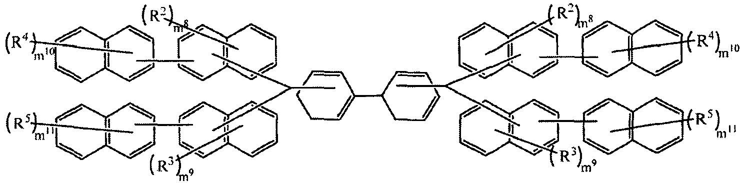

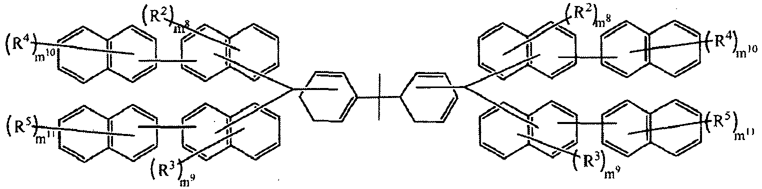

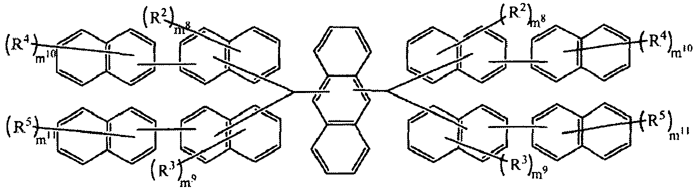

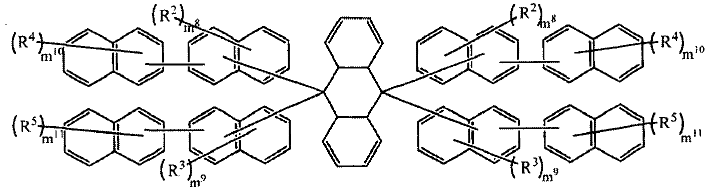

In the compounds, R² to R⁵ are the same as those described in the formula (1), m⁸ and m⁹ are each independently an integer of 0 to 6, and m¹⁰ and m¹¹ are each independently an integer of 0 to 7, provided that m¹⁰ and m¹¹ are not 0 at the same time.

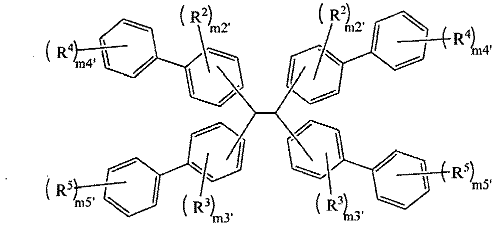

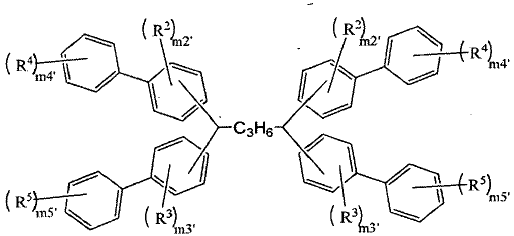

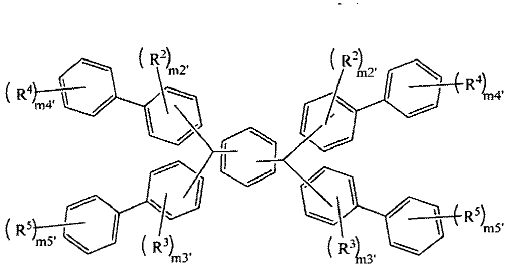

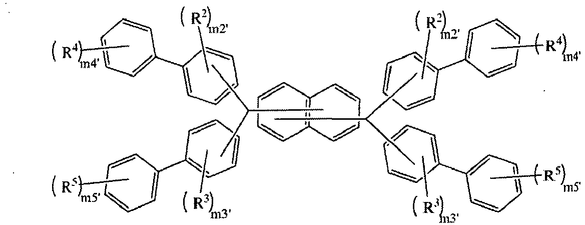

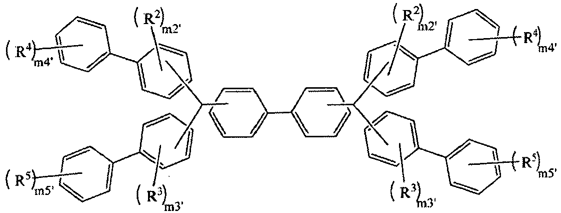

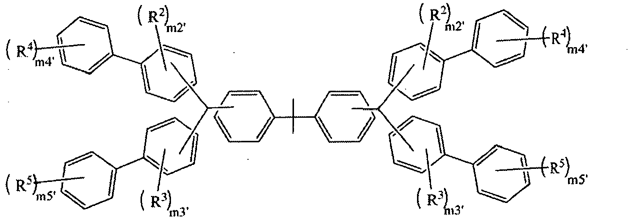

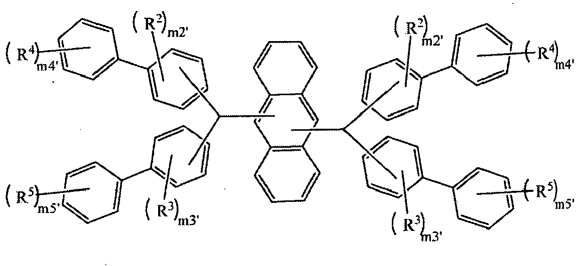

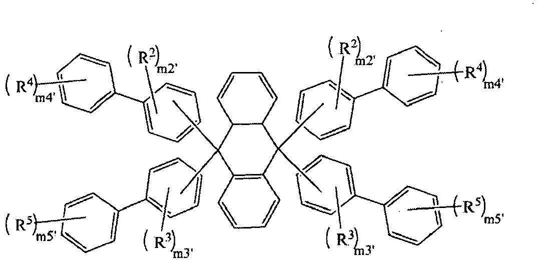

In the compounds, R² to R⁵ are the same as those described in the formula (1). m^{2'} and m^{3'} are each independently an integer of 0 to 4, and m^{4'} and m^{5'} are each independently an integer of 0 to 5, provided that m^{4'} and m^{5'} are not 0 at the same time.

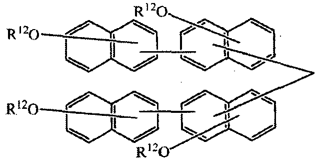

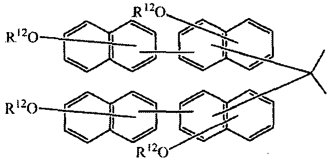

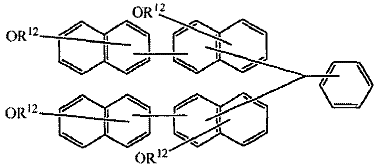

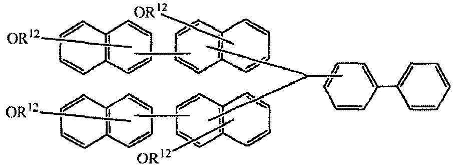

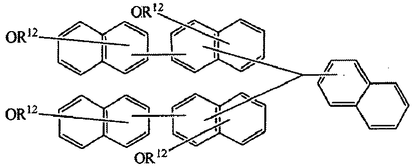

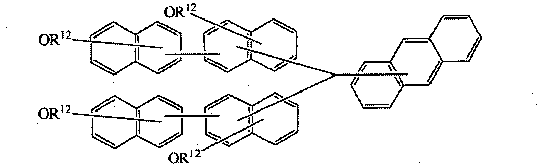

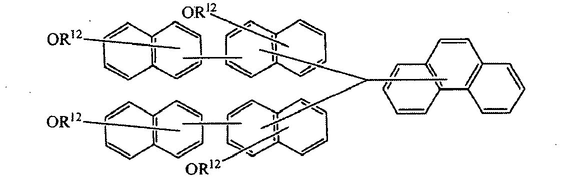

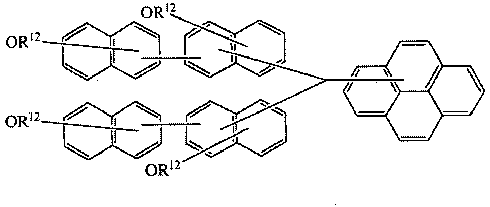

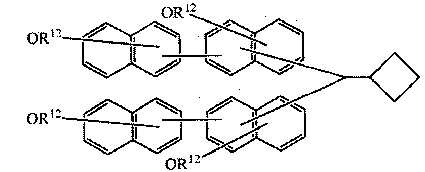

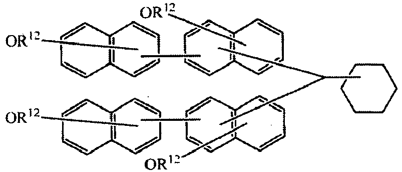

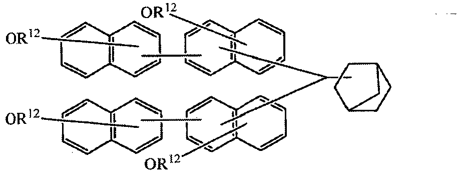

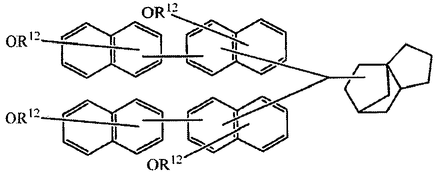

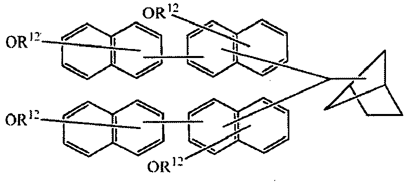

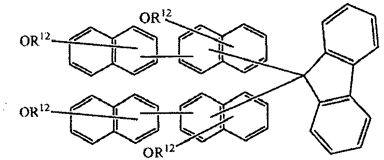

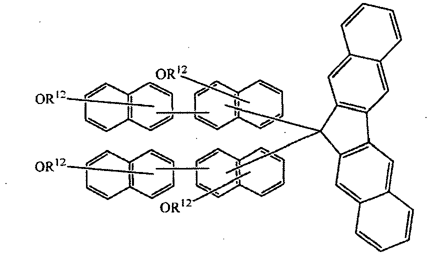

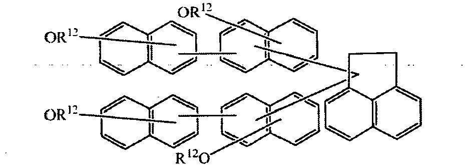

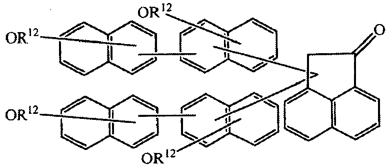

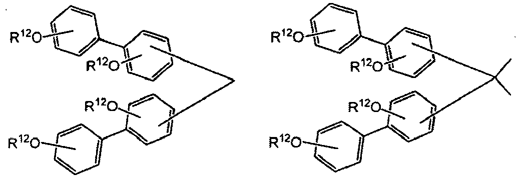

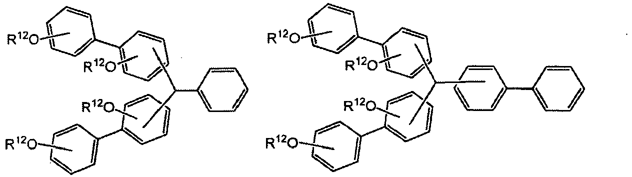

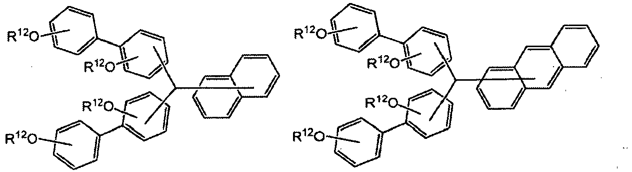

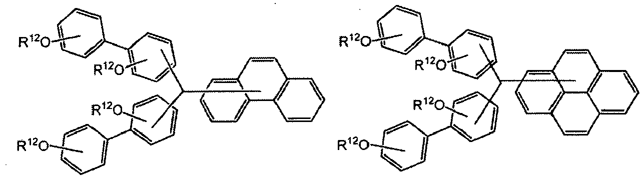

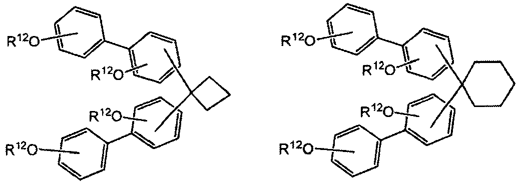

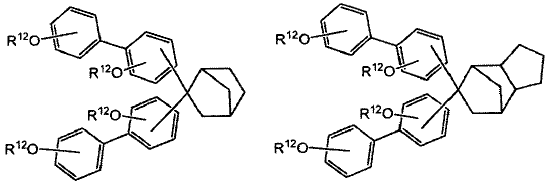

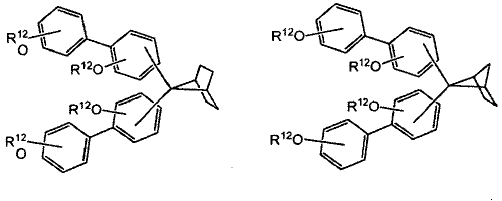

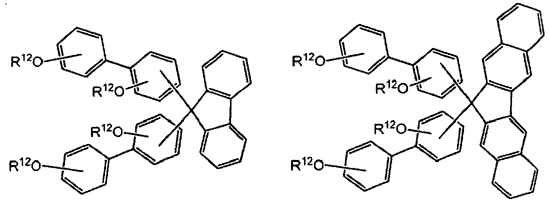

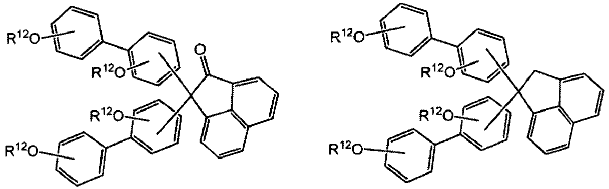

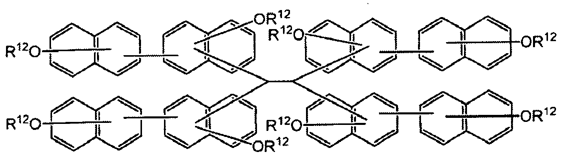

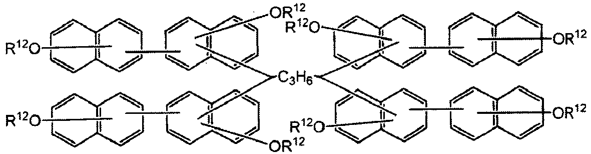

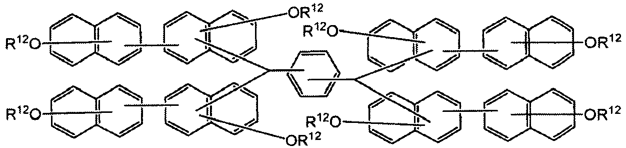

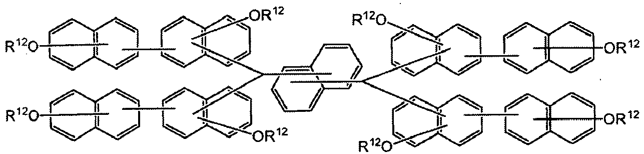

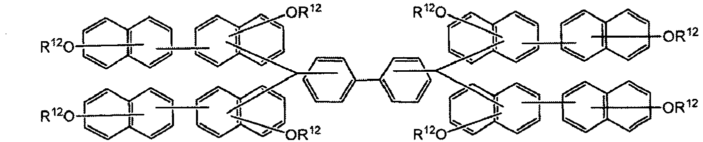

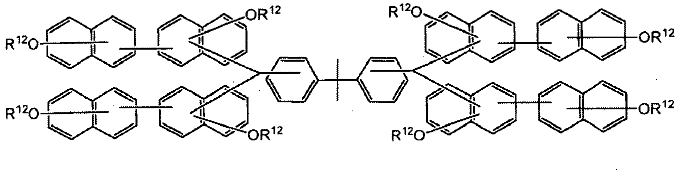

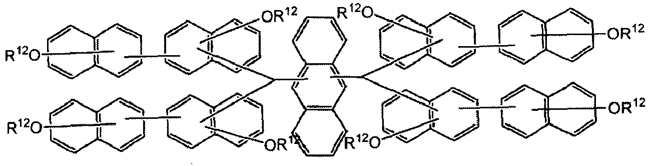

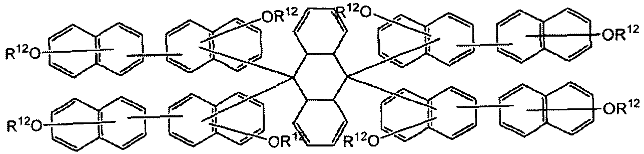

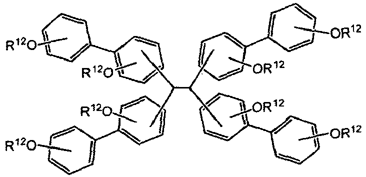

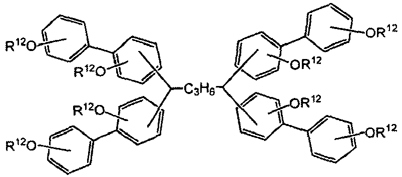

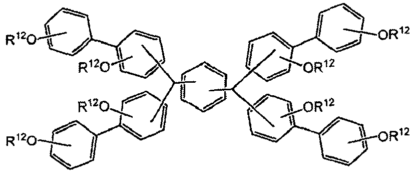

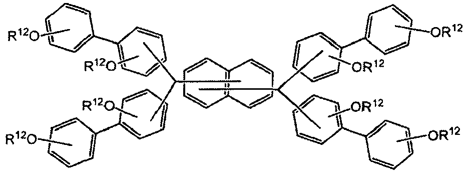

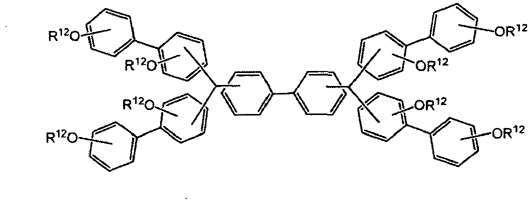

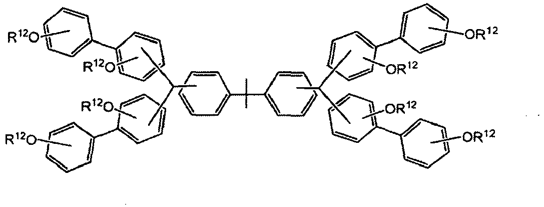

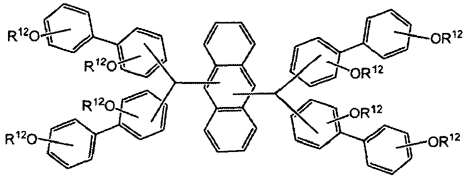

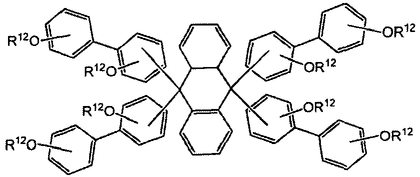

In the compounds, R¹² is the same as that described in the formula (BiF-1-CH).

The compound represented by the formula (1) for use in the present invention can be appropriately synthesized by applying a known method, and a synthesis method thereof is not particularly limited. For example, biphenols, bithiophenols, binaphthols, bithionaphthols or bianthraceneol and corresponding aldehydes or ketones can be subjected to a polycondensation reaction under ordinary pressure in the presence of an acid catalyst to thereby provide a compound (a compound where at least one R⁴ and/or at least one R⁵ to represent an alkoxy group having 1 to 30 carbon atoms in the formula (1) represents a hydroxyl group) serving as a precursor of the compound represented by the formula (1). The reaction can also be performed under pressure, if necessary.

Examples of the biphenols include biphenol, methylbiphenol and methoxybinaphthol, but are not particularly limited thereto. These can be used singly or in combinations of two or more thereof. Among them, biphenol is more preferably used in terms of stable raw material supply property.

Examples of the bithiophenols include bithiophenol, methylbithiophenol and methoxybithiophenol, but are not particularly limited thereto. These can be used singly or in combinations of two or more thereof. Among them, bithiophenol is more preferably used in terms of stable raw material supply property.

Examples of the binaphthols include binaphthol, methylbinaphthol and methoxybinaphthol, but are not particularly limited thereto. These can be used singly or in combinations of two or more thereof. Among them, binaphthol is more preferably used from the viewpoints of an increase in carbon atom concentration and an enhancement in heat resistance.

Examples of the bithionaphthols include bithionaphthol, methylbithionaphthol and methoxybithionaphthol, but are not particularly limited thereto. These can be used singly or in combinations of two or more thereof. Among them, bithionaphthol is more preferably used from the viewpoints of an increase in carbon atom concentration and an enhancement in heat resistance.

Examples of the aldehydes include formaldehyde, trioxane, paraformaldehyde, acetaldehyde, propylaldehyde, butylaldehyde, hexylaldehyde, decylaldehyde, undecylaldehyde, phenylacetaldehyde, phenylpropylaldehyde, furfural, benzaldehyde, hydroxybenzaldehyde, fluorobenzaldehyde, chlorobenzaldehyde, nitrobenzaldehyde, methylbenzaldehyde, dimethylbenzaldehyde, ethylbenzaldehyde, propylbenzaldehyde, butylbenzaldehyde, cyclohexylbenzaldehyde, benzaldehyde, hydroxybenzaldehyde, fluorobenzaldehyde, chlorobenzaldehyde, nitrobenzaldehyde, methylbenzaldehyde, dimethylbenzaldehyde, ethylbenzaldehyde, propylbenzaldehyde, butylbenzaldehyde, cyclohexylbenzaldehyde, biphenylaldehyde, naphthaldehyde, anthracenecarboxaldehyde, phenanthrenecarboxaldehyde, pyrenecarboxaldehyde, glyoxal, glutaraldehyde, phthalaldehyde, naphthalenedicarboxaldehyde, biphenyldicarboxaldehyde, anthracenedicarboxaldehyde, bis(diformylphenyl)methane, bis(diformylphenyl)propane or benzenetricarboxaldehyde is preferably used from the viewpoint of imparting a high heat resistance.

Examples of the ketones include acetone, methyl ethyl ketone, cyclobutanone, cyclopentanone, cyclohexanone, norbornanone, tricyclohexanone, tricyclodecanone, adamantanone, fluorenone, benzofluorenone, acenaphthenequinone, acenaphthenone, and anthraquinone, but are not particularly limited thereto. These can be used alone, or two or more thereof can be used in combination. Among them, cyclopentanone, cyclohexanone, norbornanone, tricyclohexanone, tricyclodecanone, adamantanone, fluorenone, benzofluorenone, acenaphthenequinone, acenaphthenone, or anthraquinone is preferably used from the viewpoint of imparting a high heat resistance.

The acid catalyst for use in the above reaction can be appropriately selected from known ones and used, and is not particularly limited. Such an acid catalyst is an inorganic acid or an organic acid, as widely known, and examples thereof include inorganic acids such as hydrochloric acid, sulfuric acid, phosphoric acid, hydrobromic acid, or hydrofluoric acid, organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, or naphthalenedisulfonic acid, Lewis acids such as zinc chloride, aluminum chloride, iron chloride, or boron trifluoride, or solid acids such as tungstosilicic acid, tungstophosphoric acid, silicomolybdic acid, or phosphomolybdic acid, but are not particularly limited thereto. Among them, organic acids and solid acids are preferable in terms of production, and hydrochloric acid or sulfuric acid is preferably used in terms of production such as availability or handleability. Herein, these acid catalysts can be used alone, or two or more thereof can be used in combination. In addition, the amount of the acid catalyst to be used can be appropriately set depending on the types of raw materials to be used and the catalyst to be used, reaction conditions, and the like, and is not particularly limited, but the amount is preferably 0.01 to 100 parts by mass based on 100 parts by mass of reaction raw materials.

A reaction solvent may also be used during the above reaction. The reaction solvent that can be used is not particularly limited and is appropriately selected from known ones, as long as the reaction of the aldehydes or ketones to be used and the biphenols, bithiophenols, binaphthols, bithionaphthols or bianthracene diol to be used progresses. Examples thereof include water, methanol, ethanol, propanol, butanol, tetrahydrofuran, dioxane, ethylene glycol dimethyl ether, ethylene glycol diethyl ether, or a mixed solvent thereof. Herein, such a solvent can be used singly or in combinations of two or more thereof.

In addition, the amount of such a solvent to be used can be appropriately set depending on the types of raw materials to be used and the catalyst to be used, reaction conditions, and the like, and is not particularly limited, but the amount is preferably in the range from 0 to 2000 parts by mass based on 100 parts by mass of reaction raw materials. Furthermore, the reaction temperature in the above reaction can be appropriately selected depending on the reactivity of reaction raw materials, and is not particularly limited, but is usually in the range from 10 to 200°C.

In order to obtain a compound serving as a precursor of the compound represented by the formula (1) of the present embodiment, the reaction temperature is preferably high and, specifically, preferably ranges from 60 to 200°C. Herein, the reaction method that can be used is appropriately selected from known methods, and is not particularly limited, but includes a method in which the biphenols, bithiophenols, binaphthols, bithionaphthols or bianthracene diol, the aldehydes or ketones, and the catalyst are charged at once, and a method in which the biphenols, bithiophenols, binaphthols, bithionaphthols or bianthracene diol, and the aldehydes or ketones are dropped in the presence of the catalyst. After completion of the polycondensation reaction, the resulting compound can be isolated according to an ordinary method, and the isolation method is not particularly limited. For example, in order to remove the unreacted raw materials and the catalyst present in the system, a common method in which the temperature in a reaction tank is raised to 130 to 230°C to remove a volatile content at about 1 to 50 mmHg can be adopted to thereby provide an objective compound.

The reaction progresses under a preferable reaction condition in which 1.0 mol to an excess amount of the biphenols, bithiophenols, binaphthols, bithionaphthols or bianthracene diol and 0.001 to 1 mol of the acid catalyst are used based on 1 mol of the aldehydes or ketones at ordinary pressure and at 50 to 150°C for about 20 minutes to 100 hours.

After completion of the reaction, the objective compound can be isolated by a known method. For example, the objective compound, a compound serving as a precursor of the compound represented by the formula (1), can be obtained by concentrating a reaction liquid, adding pure water to thereby precipitate a reaction product, cooling the resultant to room temperature followed by filtration for separation, collecting the resulting solid by filtration and drying it, then separating the solid into the reaction product and a by-product for purification by column chromatography, and performing distilling off of the solvent, filtration and drying.

The objective compound, the compound represented by the formula (1), can be obtained by, for example, subjecting the resulting precursor compound to substitution of a hydrogen atom of a phenolic hydroxyl group with a monovalent group having 1 to 30 carbon atoms by a known method.

The method for substituting a hydrogen atom of a phenolic hydroxyl group with a monovalent group having 1 to 30 carbon atoms is not particularly limited, and for example, a dehydrohalogenation reaction in which a halogenated hydrocarbon compound is reacted with the precursor compound in the presence of a basic catalyst can be performed.

The halogenated hydrocarbon compound is not particularly limited, and a halogenated hydrocarbon compound having 1 to 30 carbon atoms is suitably used. The halogenated hydrocarbon compound includes a straight hydrocarbon group, a branched hydrocarbon group, an alicyclic hydrocarbon group, an aromatic hydrocarbon group and a group including a combination of two or more thereof, as well as a halogen atom. Herein, the alicyclic hydrocarbon group also includes a bridged alicyclic hydrocarbon group. The halogenated hydrocarbon compound may also have a double bond, a hetero atom, or other halogen atom.

Examples of the halogenated hydrocarbon compound include methyl chloride, methyl bromide, methyl iodide, propyl chloride, propyl bromide, propyl iodide, butyl chloride, butyl bromide, butyl iodide, heptyl chloride, heptyl bromide, heptyl iodide, hexyl chloride, hexyl bromide, hexyl iodide, decyl chloride, decyl bromide, decyl iodide, and a compound selected from the group including those represented by the following formula (5), but are not particularly limited thereto. These can be used singly or in combinations of two or more thereof. 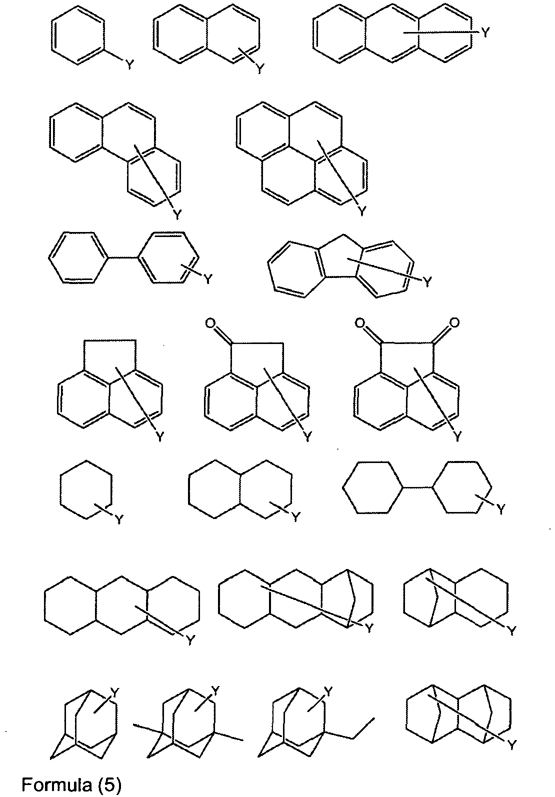 (in the formula (5), Y represents a chlorine atom, a bromine atom, or an iodine atom.)

In order to convert at least one phenolic hydroxyl group in the precursor compound to an alkoxyl group, for example, a reaction of 0.1 to 10 mol of the halogenated hydrocarbon compound based on 1 mol of the precursor compound can be performed in an organic solvent such as dimethylformamide in the presence of a basic catalyst (sodium carbonate, potassium carbonate, triethylamine, ammonia, sodium hydroxide or the like) at 0 to 150°C for about 0.5 to 20 hours. The reaction can allow at least one phenolic hydroxyl group in the precursor compound to be converted to an alkoxyl group. Then, the resultant can be subjected to separation by filtration, washing with alcohols such as methanol, washing with water, and filtration, and thereafter drying, to thereby provide the compound represented by the formula (1).

In addition, the compound represented by the formula (1) for use in the present embodiment can also be obtained by introducing an alkoxy group to the biphenols or the like and thereafter reacting the resultant with the aldehydes or ketones, and a synthesis method thereof is not particularly limited.

The compound represented by the formula (1) can be used as a material for forming an underlayer film for lithography, as it is. In addition, the compound can also be used as a resin obtained with the compound represented by the formula (1) as a monomer. For example, the compound can also be used as a resin obtained by reacting the compound represented by the formula (1) with a compound having crosslinking reactivity. Examples of the resin obtained with the compound represented by the formula (1) as a monomer include those having a structure represented by the following formula (2). That is, a material for forming an underlayer film for lithography of the present embodiment may also be one containing a resin having a structure represented by the following formula (2).

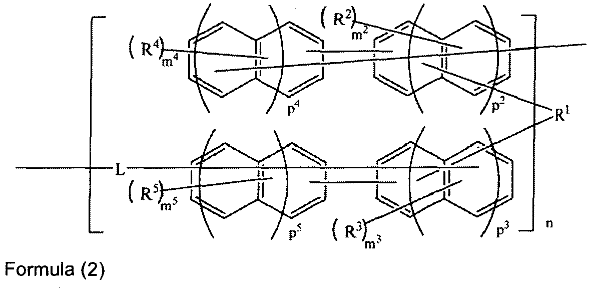

In formula (2), R¹ to R⁵, m² to m⁵, n, and p² to p⁵ are the same as those described in the formula (1).

In formula (2), L represents a straight or branched alkylene group having 1 to 20 carbon atoms, or a single bond.

The compound having crosslinking reactivity is not particularly limited as long as it can provide an oligomer or a polymer of the compound represented by the formula (1), and known one can be used therefor without any limitation. Specific examples of the compound having crosslinking reactivity include aldehyde, ketone, carboxylic acid, carboxylic halide, a halogen-containing compound, an amino compound, an imino compound, isocyanate, and an unsaturated hydrocarbon group-containing compound, but are not particularly limited thereto.

Specific examples of the resin having the structure represented by the formula (2) include, a novolac resin obtained by a condensation reaction and the like of the compound represented by the formula (1) with an aldehyde as the compound having crosslinking reactivity.

Herein, examples of the aldehyde for use in forming the novolac resin of the compound represented by the formula (1) include formaldehyde, trioxane, paraformaldehyde, benzaldehyde, acetaldehyde, propylaldehyde, phenylacetaldehyde, phenylpropylaldehyde, hydroxybenzaldehyde, chlorobenzaldehyde, nitrobenzaldehyde, methylbenzaldehyde, ethylbenzaldehyde, butylbenzaldehyde, biphenylaldehyde, naphthaldehyde, anthracenecarbaldehyde, phenanthrenecarbaldehyde, pyrenecarbaldehyde, and furfural, but are not particularly limited thereto. Among them, formaldehyde is more preferable. Herein, these aldehydes can be used alone, or two or more thereof can be used in combination. In addition, the amount of the aldehydes to be used is not particularly limited, but the amount is preferably 0.2 to 5 mol and more preferably 0.5 to 2 mol based on 1 mol of the compound represented by the formula (1).

A catalyst can also be used in the condensation reaction of the compound represented by the formula (1) with an aldehyde. The acid catalyst that can be here used is appropriately selected from known ones, and is not particularly limited. Such an acid catalyst is an inorganic acid or an organic acid, as widely known, and examples thereof include inorganic acids such as hydrochloric acid, sulfuric acid, phosphoric acid, hydrobromic acid, or hydrofluoric acid, organic acids such as oxalic acid, malonic acid, succinic acid, adipic acid, sebacic acid, citric acid, fumaric acid, maleic acid, formic acid, p-toluenesulfonic acid, methanesulfonic acid, trifluoroacetic acid, dichloroacetic acid, trichloroacetic acid, trifluoromethanesulfonic acid, benzenesulfonic acid, naphthalenesulfonic acid, or naphthalenedisulfonic acid, Lewis acids such as zinc chloride, aluminum chloride, iron chloride, or boron trifluoride, or solid acids such as tungstosilicic acid, tungstophosphoric acid, silicomolybdic acid, or phosphomolybdic acid, but are not particularly limited thereto. Among them, organic acids and solid acids are preferable in terms of production, and hydrochloric acid or sulfuric acid is preferably used in terms of production such as availability or handleability. Herein, these acid catalysts can be used alone, or two or more thereof can be used in combination. In addition, the amount of the acid catalyst to be used can be appropriately set depending on the types of raw materials to be used and the catalyst to be used, reaction conditions, and the like, and is not particularly limited, but the amount is preferably 0.01 to 100 parts by mass based on 100 parts by mass of reaction raw materials. Herein, in the case of copolymerization with a compound having a non-conjugated double bond, such as indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, 5-vinylnorborna-2-ene, α-pinene, β-pinene, and limonene, however, aldehydes are not necessarily required.

A reaction solvent can also be used in the condensation reaction of the compound represented by the formula (1) with an aldehyde. The reaction solvent in the polycondensation, which can be used, is appropriately selected from known ones, and is not particularly limited, but examples thereof include water, methanol, ethanol, propanol, butanol, tetrahydrofuran, dioxane, or a mixed solvent thereof. Herein, these solvents can be used alone, or two or more thereof can be used in combination.

In addition, the amount of the solvent to be used can be appropriately set depending on the types of raw materials to be used and the catalyst to be used, reaction conditions, and the like, and is not particularly limited, but is preferably in the range from 0 to 2000 parts by mass based on 100 parts by mass of reaction raw materials. Furthermore, the reaction temperature can be appropriately selected depending on the reactivity of reaction raw materials, and is not particularly limited, but usually ranges from 10 to 200°C. Herein, the reaction method that can be used is appropriately selected from known methods, and is not particularly limited, but includes a method in which the compound represented by the formula (1), the aldehydes, and the catalyst are charged at once, and a method in which the compound represented by the formula (1) and the aldehydes are dropped in the presence of the catalyst.

After completion of the polycondensation reaction, the resulting compound can be isolated according to an ordinary method, and the isolation method is not particularly limited. For example, in order to remove the unreacted raw materials and the catalyst present in the system, a common method in which the temperature in a reaction tank is raised to 130 to 230°C to remove a volatile content at about 1 to 50 mmHg can be adopted to thereby provide an objective novolac resin.

Herein, the resin having the structure represented by the formula (2) may be a homopolymer of the compound represented by the formula (1), or may be a copolymer with other phenols. Examples of the copolymerizable phenols include phenol, cresol, dimethylphenol, trimethylphenol, butylphenol, phenylphenol, diphenylphenol, naphthylphenol, resorcinol, methylresorcinol, catechol, butylcatechol, methoxyphenol, methoxyphenol, propylphenol, pyrogallol, and thymol, but are not particularly limited thereto.

In addition, the resin having the structure represented by the formula (2) may be one obtained by copolymerization with a polymerizable monomer other than the above-described other phenols. Examples of such a copolymerizable monomer include naphthol, methylnaphthol, methoxynaphthol, dihydroxynaphthalene, indene, hydroxyindene, benzofuran, hydroxyanthracene, acenaphthylene, biphenyl, bisphenol, trisphenol, dicyclopentadiene, tetrahydroindene, 4-vinylcyclohexene, norbornadiene, vinylnorbornaene, pinene, and limonene, but are not particularly limited thereto. Herein, the resin having the structure represented by the formula (2) may be a bi or higher functional (for example, bi to tetra) copolymer of the compound represented by the formula (1) with the above-described phenols, may be a bi or higher functional (for example, bi to tetra) copolymer of the compound represented by the formula (1) with the above-described copolymerizable monomer, or may be a ter or higher (for example, ter to tetra) copolymer of the compound represented by the formula (1) with the above-described phenols and the above-described copolymerizable monomer.

Herein, the molecular weight of the resin having the structure represented by the formula (2) is not particularly limited, and the weight average molecular weight (Mw) in terms of polystyrene is preferably 500 to 30,000, and more preferably 750 to 20,000. In addition, the resin having the structure represented by the formula (2) preferably has a dispersity (weight average molecular weight Mw/number average molecular weight Mn) in a range from 1.2 to 7, from the viewpoints of improving a crosslinking efficiency and suppressing a volatile component during baking. Herein, the Mn can be determined by a method in Examples described later.

The compound having the structure represented by the formula (1), and the resin obtained with the compound as a monomer, for example, the resin having the structure represented by the formula (2), preferably have a high solubility in the solvent from the viewpoint of making the application of a wet process easier. More specifically, the compound and/or the resin, when 1-methoxy-2-propanol (PGME) and/or propylene glycol monomethyl ether acetate (PGMEA) are/is used for the solvent, preferably have/has a solubility of 10% by mass or more in the solvent. Herein, the solubility in PGME and/or PGMEA is defined as "Mass of compound or resin/(Mass of compound or resin + Mass of solvent) × 100(% by mass)". For example, in the case where 10 g of the compound or the resin is dissolved in 90 g of PGMEA, the solubility of the resin in PGMEA is "10% by mass or more", and in the case where 10 g of the compound or the resin is not dissolved, the solubility is "less than 10% by mass".

### [Material for forming underlayer film for lithography]

A material for forming an underlayer film for lithography of the present embodiment contains at least one substance selected from the group consisting of the compound represented by the formula (1) and the resin obtained with the compound as a monomer.

In the present embodiment, the content of the substance in the material for forming an underlayer film for lithography is preferably 25 to 100% by mass, more preferably 50 to 100% by mass, further preferably 75 to 100% by mass, particularly preferably 100% by mass.

Herein, the material for forming an underlayer film for lithography of the present embodiment may include a known material for forming an underlayer film for lithography, or the like, as long as the effect of the present invention is not impaired.

### [Composition for forming underlayer film for lithography]

A composition for forming an underlayer film for lithography of the present embodiment contains the material for forming an underlayer film for lithography, and a solvent.

### [Solvent]

As the solvent for use in the present embodiment, a known solvent can be appropriately used as long as it can dissolve at least the material for forming an underlayer film for lithography. Specific examples of the solvent include ketone-based solvents such as acetone, methyl ethyl ketone, methyl isobutyl ketone and cyclohexanone, cellosolve-based solvents such as propylene glycol monomethyl ether and propylene glycol monomethyl ether acetate (PGMEA), ester-based solvents such as ethyl lactate, methyl acetate, ethyl acetate, butyl acetate, isoamyl acetate, ethyl lactate, methyl methoxypropionate and methyl hydroxyisobutyrate, alcohol-based solvents such as methanol, ethanol, isopropanol and 1-ethoxy-2-propanol (PGME), and aromatic hydrocarbons such as toluene, xylene and anisole, but are not particularly limited thereto. These solvents can be used singly or in combinations of two or more thereof.

Among the solvents, particularly preferable are cyclohexanone, propylene glycol monomethyl ether, propylene glycol monomethyl ether acetate, ethyl lactate, methyl hydroxyisobutyrate, 1-methoxy-2-propanol, and anisole, in terms of safety.

The content of the solvent is not particularly limited, but is preferably 100 to 10,000 parts by mass, more preferably 200 to 5,000 parts by mass, further preferably 300 to 1,000 parts by mass based on 100 parts by mass of the material for forming an underlayer film from the viewpoints of solubility and film formation.

The composition for forming an underlayer film for lithography of the present embodiment may include, if necessary, any component such as a crosslinking agent and an acid generator. Hereinafter, these optional components will be described.

### [Crosslinking agent]

The composition for forming an underlayer film for lithography of the present embodiment may contain, if necessary, a crosslinking agent from the viewpoint of suppression of intermixing, and the like. Specific examples of the crosslinking agent usable in the present embodiment include a melamine compound, a guanamine compound, a glycoluril compound or a urea compound, an epoxy compound, a thioepoxy compound, an isocyanate compound, an azide compound, and a compound including a double bond such as an alkenyl ether group, these compounds being substituted with at least one group selected from a methylol group, an alkoxymethyl group and an acyloxymethyl group, but are not particularly limited thereto. Herein, these crosslinking agents can be used singly or in combinations of two or more thereof. In addition, such a crosslinking agent may also be used as an additive, or may also be introduced as a pendant group into a polymer side chain. A compound including a hydroxy group can also be used as the crosslinking agent.

Specific examples of the melamine compound include hexamethylolmelamine, hexamethoxymethylmelamine, a compound in which 1 to 6 methylol groups in hexamethylolmelamine are methoxymethylated, or mixtures thereof, and hexamethoxyethylmelamine, hexaacyloxymethylmelamine, a compound in which 1 to 6 methylol groups in hexamethylolmelamine are acyloxymethylated, or mixtures thereof. Specific examples of the epoxy compound include tris(2,3-epoxypropyl)isocyanurate, trimethylolmethane triglycidyl ether, trimethylolpropane triglycidyl ether, and triethylolethane triglycidyl ether.

Specific examples of the guanamine compound include tetramethylolguanamine, tetramethoxymethylguanamine, a compound in which 1 to 4 methylol groups in tetramethylolguanamine are methoxymethylated, or mixtures thereof, and tetramethoxyethylguanamine, tetraacyloxyguanamine, a compound in which 1 to 4 methylol groups in tetramethylolguanamine are acyloxymethylated, or mixtures thereof. Specific examples of the glycoluril compound include tetramethylolglycoluril, tetramethoxyglycoluril, tetramethoxymethylglycoluril, a compound in which 1 to 4 methylol groups in tetramethylolglycoluril are methoxymethylated, or mixtures thereof, and a compound in which 1 to 4 methylol groups in tetramethylolglycoluril are acyloxymethylated, or mixtures thereof. Specific examples of the urea compound include tetramethylolurea, tetramethoxymethylurea, a compound in which 1 to 4 methylol groups in tetramethylolurea are methoxymethylated, or mixtures thereof, and tetramethoxyethylurea.

Specific examples of the compound including an alkenyl ether group include ethylene glycol divinyl ether, triethylene glycol divinyl ether, 1,2-propanediol divinyl ether, 1,4-butanediol divinyl ether, tetramethylene glycol divinyl ether, neopentyl glycol divinyl ether, trimethylolpropane trivinyl ether, hexanediol divinyl ether, 1,4-cyclohexanediol divinyl ether, pentaerythritol trivinyl ether, pentaerythritol tetravinyl ether, sorbitol tetravinyl ether, sorbitol pentavinyl ether, and trimethylolpropane trivinyl ether.

The content of the crosslinking agent in the composition for forming an underlayer film for lithography of the present embodiment is not particularly limited, but is preferably 5 to 50 parts by mass, more preferably 10 to 40 parts by mass, further preferably 15 to 35 parts by mass based on 100 parts by mass of the material for forming an underlayer film for lithography. The content is set within the range to result in tendencies to suppress the occurrence of the mixing phenomenon with the resist layer, and to result in tendencies to enhance an antireflective effect and improve film formability after crosslinking.

### [Acid Generator]

The composition for forming an underlayer film for lithography of the present embodiment may also contain, if necessary, an acid generator from the viewpoint of further promoting a crosslinking reaction by heat. As the acid generator in the art, one for generating an acid by pyrolysis and one for generating an acid by light irradiation are known, and any of them can be used.

The acid generator includes:
1) an onium salt of the following general formula (P1a-1), (P1a-2), (P1a-3) or (P1b),
2) a diazomethane derivative of the following general formula (P2),
3) a glyoxime derivative of the following general formula (P3),
4) a bissulfone derivative of the following general formula (P4),
5) a sulfonic acid ester of an N-hydroxyimide compound of the following general formula (P5),
6) a β-ketosulfonic acid derivative,
7) a disulfone derivative,
8) a nitrobenzylsulfonate derivative, and
9) a sulfonic acid ester derivative, but is not particularly limited thereto. Herein, these acid generators can be used alone, or two or more thereof can be used in combination.

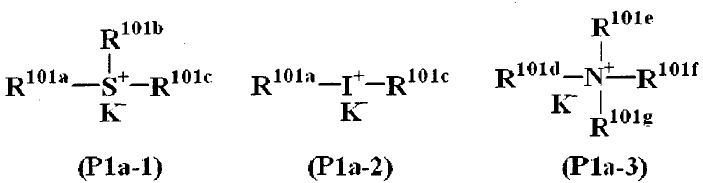

In the above formulae, each of R^{101a}, R^{101b} and R^{101c} independently represents a straight, branched or cyclic alkyl group, alkenyl group, oxoalkyl group or oxoalkenyl group having 1 to 12 carbon atoms, an aryl group having 6 to 20 carbon atoms, or an aralkyl group or aryloxoalkyl group having 7 to 12 carbon atoms, and a part or all of hydrogen atoms of these groups may be substituted with an alkoxy group or the like. In addition, R^{101b} and R^{101c} may form a ring, and if forming a ring, each of R^{101b} and R^{101c} independently represents an alkylene group having 1 to 6 carbon atoms. K⁻ represents a non-nucleophilic counter ion. R^{101d}, R^{101e}, R^{101f} and R^{101g} are represented by each independently adding a hydrogen atom to R^{101a}, R^{101b} and R^{101c}. R^{101d} and R^{101e}, and R^{101d}, R^{101e} and R^{101f} may form a ring, and if forming a ring, R^{101d} and R^{101e}, and R^{101d}, R^{101e} and R^{101f} represent an alkylene group having 3 to 10 carbon atoms, or a heteroaromatic ring having therein the nitrogen atom(s) in the formula.

R^{101a}, R^{101b}, R^{101c}, R^{101d}, R^{101e}, R^{101f} and R^{101g} described above may be the same or different from one another. Specifically, examples of the alkyl group include a methyl group, an ethyl group, a propyl group, an isopropyl group, a n-butyl group, a sec-butyl group, a tert-butyl group, a pentyl group, a hexyl group, a heptyl group, an octyl group, a cyclopentyl group, a cyclohexyl group, a cycloheptyl group, a cyclopropylmethyl group, a 4-methylcyclohexyl group, a cyclohexylmethyl group, a norbornyl group and an adamantyl group. Examples of the alkenyl group include a vinyl group, an allyl group, a propenyl group, a butenyl group, a hexenyl group and a cyclohexenyl group. Examples of the oxoalkyl group include a 2-oxocyclopentyl group and a 2-oxocyclohexyl group, and can include a 2-oxopropyl group, a 2-cyclopentyl-2-oxoethyl group, a 2-cyclohexyl-2-oxoethyl group and a 2-(4-methylcyclohexyl)-2-oxoethyl group. Examples of the oxoalkenyl group include a 2-oxo-4-cyclohexenyl group and a 2-oxo-4-propenyl group. Examples of the aryl group include a phenyl group and a naphthyl group, alkoxyphenyl groups such as a p-methoxyphenyl group, a m-methoxyphenyl group, an o-methoxyphenyl group, an ethoxyphenyl group, a p-tert-butoxyphenyl group and a m-tert-butoxyphenyl group, alkylphenyl groups such as a 2-methylphenyl group, a 3-methylphenyl group, a 4-methylphenyl group, an ethylphenyl group, a 4-tert-butylphenyl group, a 4-butylphenyl group and a dimethylphenyl group, alkylnaphthyl groups such as a methylnaphthyl group and an ethylnaphthyl group, alkoxynaphthyl groups such as a methoxynaphthyl group and an ethoxynaphthyl group, dialkylnaphthyl groups such as a dimethylnaphthyl group and a diethylnaphthyl group, and dialkoxynaphthyl groups such as a dimethoxynaphthyl group and a diethoxynaphthyl group. Examples of the aralkyl group include a benzyl group, a phenylethyl group and a phenethyl group. Examples of the aryloxoalkyl group include 2-aryl-2-oxoethyl groups such as a 2-phenyl-2-oxoethyl group, a 2-(1-naphthyl)-2-oxoethyl group and a 2-(2-naphthyl)-2-oxoethyl group. Examples of the non-nucleophilic counter ion, K⁻, include halide ions such as a chloride ion and a bromide ion, fluoroalkyl sulfonates such as triflate, 1,1,1-trifluoroethane sulfonate and nonafluorobutane sulfonate, aryl sulfonates such as tosylate, benzene sulfonate, 4-fluorobenzene sulfonate and 1,2,3,4,5-pentafluorobenzene sulfonate, and alkyl sulfonates such as mesylate and butane sulfonate.

In the case where R^{101d}, R^{101e}, R^{101f} and R^{101g} are each a heteroaromatic ring having the nitrogen atom(s) in the formula, examples of the heteroaromatic ring include imidazole derivatives (for example, imidazole, 4-methylimidazole, and 4-methyl-2-phenylimidazole), pyrazole derivatives, furazan derivatives, pyrroline derivatives (for example, pyrroline and 2-methyl-1-pyrroline), pyrrolidine derivatives (for example, pyrrolidine, N-methylpyrrolidine, pyrrolidinone, and N-methylpyrrolidone), imidazoline derivatives, imidazolidine derivatives, pyridine derivatives (for example, pyridine, methylpyridine, ethylpyridine, propylpyridine, butylpyridine, 4-(1-butylpentyl)pyridine, dimethylpyridine, trimethylpyridine, triethylpyridine, phenylpyridine, 3-methyl-2-phenylpyridine, 4-tert-butylpyridine, diphenylpyridine, benzylpyridine, methoxypyridine, butoxypyridine, dimethoxypyridine, 1-methyl-2-pyridone, 4-pyrrolidinopyridine, 1-methyl-4-phenylpyridine, 2-(1-ethylpropyl)pyridine, aminopyridine, and dimethylaminopyridine), pyridazine derivatives, pyrimidine derivatives, pyrazine derivatives, pyrazoline derivatives, pyrazolidine derivatives, piperidine derivatives, piperazine derivatives, morpholine derivatives, indole derivatives, isoindole derivatives, 1H-indazole derivatives, indoline derivatives, quinoline derivatives (for example, quinoline and 3-quinolinecarbonitrile), isoquinoline derivatives, cinnoline derivatives, quinazoline derivatives, quinoxaline derivatives, phthalazine derivatives, purine derivatives, pteridin derivatives, carbazole derivatives, phenanthridine derivatives, acridine derivatives, phenazine derivatives, 1,10-phenanthroline derivatives, adenine derivatives, adenosine derivatives, guanine derivatives, guanosine derivatives, uracil derivative, and uridine derivatives.

The formula (P1a-1) and the formula (P1a-2) have effects as both of a photo acid generator and a thermal acid generator, but the formula (P1a-3) acts as a thermal acid generator.

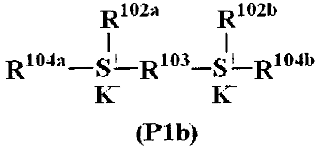

In the formula (P1b) , each of R^{102a} and R^{102b} independently represents a straight, branched or cyclic alkyl group having 1 to 8 carbon atoms. R¹⁰³ represents a straight, branched or cyclic alkylene group having 1 to 10 carbon atoms. Each of R^{104a} and R^{104b} independently represents a 2-oxoalkyl group having 3 to 7 carbon atoms. K⁻ represents a non-nucleophilic counter ion.

Specific examples of the R^{102a} and R^{102b} include a methyl group, an ethyl group, a propyl group, an isopropyl group, a n-butyl group, a sec-butyl group, a tert-butyl group, a pentyl group, a hexyl group, a heptyl group, an octyl group, a cyclopentyl group, a cyclohexyl group, a cyclopropylmethyl group, a 4-methylcyclohexyl group and a cyclohexylmethyl group. Examples of R¹⁰³ include a methylene group, an ethylene group, a propylene group, a butylene group, a pentylene group, a hexylene group, a heptylene group, an octylene group, a nonylene group, a 1,4-cyclohexylene group, a 1,2-cyclohexylene group, a 1,3-cyclopentylene group, a 1,4-cyclooctylene group and a 1,4-cyclohexanedimethylene group. Examples of R^{104a} and R^{104b} include a 2-oxopropyl group, a 2-oxocyclopentyl group, a 2-oxocyclohexyl group and a 2-oxocycloheptyl group. K⁻ includes the same as those described in the formula (P1a-1), (P1a-2) and (P1a-3).

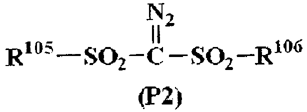

In the formula (P2), each of R¹⁰⁵ and R¹⁰⁶ independently represents a straight, branched or cyclic alkyl group or halogenated alkyl group having 1 to 12 carbon atoms, an aryl group or halogenated aryl group having 6 to 20 carbon atoms, or an aralkyl group having 7 to 12 carbon atoms.

Examples of the alkyl group in each of R¹⁰⁵ and R¹⁰⁶ include a methyl group, an ethyl group, a propyl group, an isopropyl group, a n-butyl group, a sec-butyl group, a tert-butyl group, a pentyl group, a hexyl group, a heptyl group, an octyl group, an amyl group, a cyclopentyl group, a cyclohexyl group, a cycloheptyl group, a norbornyl group, and an adamantyl group. Examples of the halogenated alkyl group include a trifluoromethyl group, a 1,1,1-trifluoroethyl group, a 1,1,1-trichloroethyl group, and a nonafluorobutyl group. Examples of the aryl group include alkoxyphenyl groups such as a phenyl group, a p-methoxyphenyl group, a m-methoxyphenyl group, an o-methoxyphenyl group, an ethoxyphenyl group, a p-tert-butoxyphenyl group, and a m-tert-butoxyphenyl group, and alkylphenyl groups such as a 2-methylphenyl group, a 3-methylphenyl group, a 4-methylphenyl group, an ethylphenyl group, a 4-tert-butylphenyl group, a 4-butylphenyl group, and a dimethylphenyl group. Examples of the halogenated aryl group include a fluorophenyl group, a chlorophenyl group, and a 1,2,3,4,5-pentafluorophenyl group. Examples of the aralkyl group include a benzyl group and a phenethyl group.

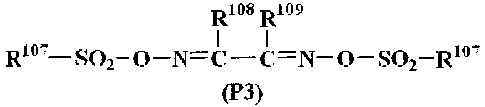

In the formula (P3), each of R¹⁰⁷, R¹⁰⁸ and R¹⁰⁹ independently represents a straight, branched or cyclic alkyl group or halogenated alkyl group having 1 to 12 carbon atoms, an aryl group or halogenated aryl group having 6 to 20 carbon atoms, or an aralkyl group having 7 to 12 carbon atoms. R¹⁰⁸ and R¹⁰⁹ may be bonded with each other to form a cyclic structure, and if forming a cyclic structure, each of R¹⁰⁸ and R¹⁰⁹ represents a straight or branched alkylene group having 1 to 6 carbon atoms.

The alkyl group, halogenated alkyl group, aryl group, halogenated aryl group, and aralkyl group in each of R¹⁰⁷, R¹⁰⁸ and R¹⁰⁹ include the same as those described in R¹⁰⁵ and R¹⁰⁶. Herein, examples of the alkylene group in each of R¹⁰⁸ and R¹⁰⁹ include a methylene group, an ethylene group, a propylene group, a butylene group, and a hexylene group. 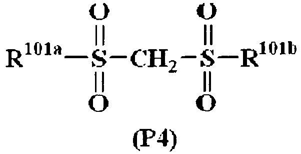 In the formula (P4), R^{101a} and R^{101b} are the same as those described above.

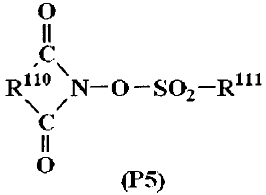

In the formula (P5), R¹¹⁰ represents an arylene group having 6 to 10 carbon atoms, an alkylene group having 1 to 6 carbon atoms, or an alkenylene group having 2 to 6 carbon atoms, and a part or all of hydrogen atoms of these groups may be further substituted with a straight or branched alkyl group or alkoxy group having 1 to 4 carbon atoms, a nitro group, an acetyl group, or a phenyl group. R¹¹¹ represents a straight, branched or substituted alkyl group, alkenyl group or alkoxyalkyl group having 1 to 8 carbon atoms, a phenyl group, or a naphthyl group, and a part or all of hydrogen atoms of these groups may be further substituted with an alkyl group or alkoxy group having 1 to 4 carbon atoms; a phenyl group that may be substituted with an alkyl group or alkoxy group having 1 to 4 carbon atoms, a nitro group, or an acetyl group; a heteroaromatic group having 3 to 5 carbon atoms; or a chlorine atom or a fluorine atom.

Herein, examples of the arylene group in R¹¹⁰ include a 1,2-phenylene group and a 1,8-naphthylene group. Examples of the alkylene group include a methylene group, an ethylene group, a trimethylene group, a tetramethylene group, a phenylethylene group, and a norbornane-2,3-diyl group. Examples of the alkenylene group include a 1,2-vinylene group, a 1-phenyl-1,2-vinylene group, and a 5-norbornene-2,3-diyl group. The alkyl group in R¹¹¹ includes the same as those in R^{101a} to R^{101c}. Examples of the alkenyl group include a vinyl group, a 1-propenyl group, an allyl group, a 1-butenyl group, a 3-butenyl group, an isoprenyl group, a 1-pentenyl group, a 3-pentenyl group, a 4-pentenyl group, a dimethylallyl group, a 1-hexenyl group, a 3-hexenyl group, a 5-hexenyl group, a 1-heptenyl group, a 3-heptenyl group, a 6-heptenyl group, and a 7-octenyl group. Examples of the alkoxyalkyl group include a methoxymethyl group, an ethoxymethyl group, a propoxymethyl group, a butoxymethyl group, a pentyloxymethyl group, a hexyloxymethyl group, a heptyloxymethyl group, a methoxyethyl group, an ethoxyethyl group, a propoxyethyl group, a butoxyethyl group, a pentyloxyethyl group, a hexyloxyethyl group, a methoxypropyl group, an ethoxypropyl group, a propoxypropyl group, a butoxypropyl group, a methoxybutyl group, an ethoxybutyl group, a propoxybutyl group, a methoxypentyl group, an ethoxypentyl group, a methoxyhexyl group, and a methoxyheptyl group.

Herein, Examples of the alkyl group having 1 to 4 carbon atoms, which may be further substituted, include a methyl group, an ethyl group, a propyl group, an isopropyl group, a n-butyl group, a an isobutyl group, and a tert-butyl group. Examples of the alkoxy group having 1 to 4 carbon atoms include a methoxy group, an ethoxy group, a propoxy group, an isopropoxy group, a n-butoxy group, an isobutoxy group, and tert-butoxy group. Examples of the phenyl group that may be substituted with an alkyl group or alkoxy group having 1 to 4 carbon atoms, a nitro group, or an acetyl group include a phenyl group, a tolyl group, a p-tert-butoxyphenyl group, a p-acetylphenyl group, and a p-nitrophenyl group. Examples of the heteroaromatic group having 3 to 5 carbon atoms include a pyridyl group and a furyl group.

Specific examples of the acid generator include onium salts such as tetramethylammonium trifluoromethanesulfonate, tetramethylammonium nonafluorobutanesulfonate, triethylammonium nonafluorobutanesulfonate, pyridinium nonafluorobutanesulfonate, triethylammonium camphorsulfonate, pyridinium camphorsulfonate, tetra n-butylammonium nonafluorobutanesulfonate, tetraphenylammonium nonafluorobutanesulfonate, tetramethylammonium p-toluenesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, bis(p-tert-butoxyphenyl)phenylsulfonium trifluoromethanesulfonate, tris(p-tert-butoxyphenyl)sulfonium trifluoromethanesulfonate, triphenylsulfonium p-toluenesulfonate, (p-tert-butoxyphenyl)diphenylsulfonium p-toluenesulfonate, bis(p-tert-butoxyphenyl)phenylsulfonium p-toluenesulfonate, tris(p-tert-butoxyphenyl)sulfonium p-toluenesulfonate, triphenylsulfonium nonafluorobutanesulfonate, triphenylsulfonium butanesulfonate, trimethylsulfonium trifluoromethanesulfonate, trimethylsulfonium p-toluenesulfonate, cyclohexylmethyl(2-oxocyclohexyl)sulfonium trifluoromethanesulfonate, cyclohexylmethyl(2-oxocyclohexyl)sulfonium p-toluenesulfonate, dimethylphenylsulfonium trifluoromethanesulfonate, dimethylphenylsulfonium p-toluenesulfonate, dicyclohexylphenylsulfonium trifluoromethanesulfonate, dicyclohexylphenylsulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl)sulfonium trifluoromethanesulfonate, ethylene bis[methyl(2-oxocyclopentyl)sulfonium trifluoromethanesulfonate], and 1,2'-naphthylcarbonylmethyltetrahydrothiophenium triflate, diazomethane derivatives such as bis(benzenesulfonyl)diazomethane, bis(p-toluenesulfonyl)diazomethane, bis(xylenesulfonyl)diazomethane, bis(cyclohexylsulfonyl)diazomethane, bis(cyclopentylsulfonyl)diazomethane, bis(n-butylsulfonyl)diazomethane, bis(isobutylsulfonyl)diazomethane, bis(sec-butylsulfonyl)diazomethane, bis(n-propylsulfonyl)diazomethane, bis(isopropylsulfonyl)diazomethane, bis(tert-butylsulfonyl)diazomethane, bis(n-amylsulfonyl)diazomethane, bis(isoamylsulfonyl)diazomethane, bis(sec-amylsulfonyl)diazomethane, bis(tert-amylsulfonyl)diazomethane, 1-cyclohexylsulfonyl-1-(tert-butylsulfonyl)diazomethane, 1-cyclohexylsulfonyl-1-(tert-amylsulfonyl)diazomethane, and 1-tert-amylsulfonyl-1-(tert-butylsulfonyl)diazomethane, glyoxime derivatives such as bis-(p-toluenesulfonyl)-α-dimethylglyoxime, bis-(p-toluesulfonyl)-α-diphenylglyoxime, bis-(p-toluenesulfonyl)-α-dicyclohexylglyoxime, bis-(p-toluenesulfonyl)-2,3-pentanedioneglyoxime, bis-(p-toluenesulfonyl)-2-methyl-3,4-pentanedioneglyoxime, bis-(n-butanesulfonyl)-α-dimethylglyoxime, bis-(n-butanesulfonyl)-α-diphenylglyoxime, bis-(n-butanesulfonyl)-α-dicyclohexylglyoxime, bis-(n-butanesulfonyl)-2,3-pentanedioneglyoxime, bis-(n-butanesulfonyl)-2-methyl-3,4-pentanedioneglyoxime, bis-(methanesulfonyl)-α-dimethylglyoxime, bis-(trifluoromethanesulfonyl)-α-dimethylglyoxime, bis-(1,1,1-trifluoroethanesulfonyl)-α-dimethylglyoxime, bis-(tert-butanesulfonyl)-α-dimethylglyoxime, bis-(perfluorooctanesulfonyl)-α-dimethylglyoxime, bis-(cyclohexanesulfonyl)-α-dimethylglyoxime, bis-(benzenesulfonyl)-α-dimethylglyoxime, bis-(p-fluorobenzenesulfonyl)-α-dimethylglyoxime, bis-(p-tert-butylbenzenesulfonyl)-α-dimethylglyoxime, bis-(xylenesulfonyl)-α-dimethylglyoxime, and bis-(camphorsulfonyl)-α-dimethylglyoxime, bissulfone derivatives, such as bisnaphthylsulfonylmethane, bistrifluoromethylsulfonylmethane, bismethylsulfonylmethane, bisethylsulfonylmethane, bispropylsulfonylmethane, bisisopropylsulfonylmethane, bis-p-toluenesulfonylmethane, and bisbenzenesulfonylmethane, β-ketosulfone derivatives such as 2-cyclohexylcarbonyl-2-(p-toluenesulfonyl)propane and 2-isopropylcarbonyl-2-(p-toluenesulfonyl)propane, disulfone derivatives such as a diphenyldisulfone derivative and a dicyclohexyldisulfone derivative, nitrobenzylsulfonate derivatives such as 2,6-dinitrobenzyl p-toluenesulfonate and 2,4-dinitrobenzyl p-toluenesulfonate, sulfonic acid ester derivatives such as 1,2,3-tris(methanesulfonyloxy)benzene, 1,2,3-tris(trifluoromethanesulfonyloxy)benzene, and 1,2,3-tris(p-toluenesulfonyloxy)benzene, and sulfonic acid ester derivatives of a N-hydroxyimide compound, such as N-hydroxysuccinimide methanesulfonic acid ester, N-hydroxysuccinimide trifluoromethanesulfonic acid ester, N-hydroxysuccinimide ethanesulfonic acid ester, N-hydroxysuccinimide 1-propanesulfonic acid ester, N-hydroxysuccinimide 2-propanesulfonic acid ester, N-hydroxysuccinimide 1-pentanesulfonic acid ester, N-hydroxysuccinimide 1-octanesulfonic acid ester, N-hydroxysuccinimide p-toluenesulfonic acid ester, N-hydroxysuccinimide p-methoxybenzenesulfonic acid ester, N-hydroxysuccinimide 2-chloroethanesulfonic acid ester, N-hydroxysuccinimide benzenesulfonic acid ester, N-hydroxysuccinimide -2,4,6-trimethylbenzenesulfonic acid ester, N-hydroxysuccinimide 1-naphthalenesulfonic acid ester, N-hydroxysuccinimide 2-naphthalenesulfonic acid ester, N-hydroxy-2-phenylsuccinimide methanesulfonic acid ester, N-hydroxymaleimide methanesulfonic acid ester, N-hydroxymaleimide ethanesulfonic acid ester, N-hydroxy-2-phenylmaleimide methanesulfonic acid ester, N-hydroxyglutarimide methanesulfonic acid ester, N-hydroxyglutarimide benzenesulfonic acid ester, N-hydroxyphthalimide methanesulfonic acid ester, N-hydroxyphthalimide benzenesulfonic acid ester, N-hydroxyphthalimide trifluoromethanesulfonic acid ester, N-hydroxyphthalimide p-toluenesulfonic acid ester, N-hydroxynaphthalimide methanesulfonic acid ester, N-hydroxynaphthalimide benzenesulfonic acid ester, N-hydroxy-5-norbornene-2,3-dicarboxyimide methanesulfonic acid ester, N-hydroxy-5-norbornene-2,3-dicarboxyimide trifluoromethanesulfonic acid ester, and N-hydroxy-5-norbornene-2,3-dicarboxyimide p-toluenesulfonic acid ester.

Among them, in particular, onium salts such as triphenylsulfonium trifluoromethanesulfonate, (p-tert-butoxyphenyl)diphenylsulfonium trifluoromethanesulfonate, tris(p-tert-butoxyphenyl)sulfonium trifluoromethanesulfonate, triphenylsulfonium p-toluenesulfonate, (p-tert-butoxyphenyl)diphenylsulfonium p-toluenesulfonate, tris(p-tert-butoxyphenyl)sulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl) sulfonium trifluoromethanesulfonate, and 1,2'-naphthylcarbonylmethyltetrahydrothiophenium triflate, diazomethane derivatives such as bis(benzenesulfonyl)diazomethane, bis(p-toluenesulfonyl)diazomethane, bis(cyclohexylsulfonyl)diazomethane, bis(n-butylsulfonyl)diazomethane, bis(isobutylsulfonyl)diazomethane, bis(sec-butylsulfonyl)diazomethane, bis(n-propylsulfonyl)diazomethane, bis(isopropylsulfonyl)diazomethane, and bis(tert-butylsulfonyl)diazomethane, glyoxime derivatives such as bis-(p-toluenesulfonyl)-α-dimethylglyoxime and bis-(n-butanesulfonyl)-α-dimethylglyoxime, bissulfone derivatives such as bisnaphthylsulfonylmethane, and sulfonic acid ester derivatives of an N-hydroxyimide compound, such as N-hydroxysuccinimide methanesulfonic acid ester, N-hydroxysuccinimide trifluoromethanesulfonic acid ester, N-hydroxysuccinimide 1-propanesulfonic acid ester, N-hydroxysuccinimide 2-propanesulfonic acid ester, N-hydroxysuccinimide 1-pentanesulfonic acid ester, N-hydroxysuccinimide p-toluenesulfonic acid ester, N-hydroxynaphthalimide methanesulfonic acid ester, and N-hydroxynaphthalimide benzenesulfonic acid ester are preferably used.

The content of the acid generator in the composition for forming an underlayer film for lithography of the present embodiment is not particularly limited, but is preferably 0.1 to 50 parts by mass, more preferably 0.5 to 40 parts by mass, further preferably 1.0 to 30 parts by mass based on 100 parts by mass of the material for forming an underlayer film for lithography. The content is set within the above range to result in a tendency to increase the amount of acid generation to promote a crosslinking reaction, and also to result in a tendency to suppress the occurrence of the mixing phenomenon with the resist layer.

Furthermore, the composition for forming an underlayer film for lithography of the present embodiment may contain a basic compound from the viewpoint of improving preservation stability.

### [Basic compound]

The basic compound serves as a quencher to an acid for preventing a trace amount of the acid generated from the acid generator from promoting a crosslinking reaction. Examples of such a basic compound include primary, secondary, and tertiary aliphatic amines, mixed amines, aromatic amines, heterocyclic amines, a nitrogen-containing compound having a carboxy group, a nitrogen-containing compound having a sulfonyl group, a nitrogen-containing compound having a hydroxyl group, a nitrogen-containing compound having a hydroxyphenyl group, an alcoholic nitrogen-containing compound, an amide derivative, and an imide derivative, but are not particularly limited thereto.

Specific examples of the primary aliphatic amines include ammonia, methylamine, ethylamine, n-propylamine, isopropylamine, n-butylamine, isobutylamine, sec-butylamine, tert-butylamine, pentylamine, tert-amylamine, cyclopentylamine, hexylamine, cyclohexylamine, heptylamine, octylamine, nonylamine, decylamine, dodecylamine, cetylamine, methylenediamine, ethylenediamine, and tetraethylenepentamine. Specific examples of the secondary aliphatic amines include dimethylamine, diethylamine, di-n-propylamine, diisopropylamine, di-n-butylamine, diisobutylamine, disec-butylamine, dipentylamine, dicyclopentylamine, dihexylamine, dicyclohexylamine, diheptylamine, dioctylamine, dinonylamine, didecylamine, didodecylamine, dicetylamine, N,N-dimethylmethylenediamine, N,N-dimethylethylenediamine, and N,N-dimethyltetraethylenepentamine. Specific examples of the tertiary aliphatic amines include trimethylamine, triethylamine, tri-n-propylamine, triisopropylamine, tri-n-butylamine, triisobutylamine, tri-sec-butylamine, tripentylamine, tricyclopentylamine, trihexylamine, tricyclohexylamine, triheptylamine, trioctylamine, trinonylamine, tridecylamine, tridodecylamine, tricetylamine, N,N,N',N'-tetramethylmethylenediamine, N,N,N',N'-tetramethylethylenediamine, and N,N,N',N'-tetramethyltetraethylenepentamine.

Specific examples of the mixed amines include dimethylethylamine, methylethylpropylamine, benzylamine, phenethylamine, and benzyldimethylamine. Specific examples of the aromatic amines and heterocyclic amines include aniline derivatives (for example, aniline, N-methylaniline, N-ethylaniline, N-propylaniline, N,N-dimethylaniline, 2-methylaniline, 3-methylaniline, 4-methylaniline, ethylaniline, propylaniline, trimethylaniline, 2-nitroaniline, 3-nitroaniline, 4-nitroaniline, 2,4-dinitroaniline, 2,6-dinitroaniline, 3,5-dinitroaniline, and N,N-dimethyltoluidine), diphenyl(p-tolyl)amine, methyldiphenylamine, triphenylamine, phenylenediamine, naphthylamine, diaminonaphthalene, pyrrole derivatives (for example, pyrrole, 2H-pyrrole, 1-methylpyrrole, 2,4-dimethylpyrrole, 2,5-dimethylpyrrole, and N-methylpyrrole), oxazole derivatives (for example, oxazole and isoxazole), thiazole derivatives (for example, thiazole and isothiazole), imidazole derivatives (for example, imidazole, 4-methylimidazole, and 4-methyl-2-phenylimidazole), pyrazole derivatives, furazan derivatives, pyrroline derivatives (for example, pyrroline and 2-methyl-1-pyrroline), pyrrolidine derivatives (for example, pyrrolidine, N-methylpyrrolidine, pyrrolidinone, and N-methylpyrrolidone), imidazoline derivatives, imidazolidine derivatives, pyridine derivatives (for example, pyridine, methylpyridine, ethylpyridine, propylpyridine, butylpyridine, 4-(1-butylpentyl)pyridine, dimethylpyridine, trimethylpyridine, triethylpyridine, phenylpyridine, 3-methyl-2-phenylpyridine, 4-tert-butylpyridine, diphenylpyridine, benzylpyridine, methoxypyridine, butoxypyridine, dimethoxypyridine, 1-methyl-2-pyridone, 4-pyrrolidinopyridine, 1-methyl-4-phenylpyridine, 2-(1-ethylpropyl)pyridine, aminopyridine, and dimethylaminopyridine), pyridazine derivatives, pyrimidine derivatives, pyrazine derivatives, pyrazoline derivatives, pyrazolidine derivatives, piperidine derivatives, piperazine derivatives, morpholine derivatives, indole derivatives, isoindole derivatives, 1H-indazole derivatives, indoline derivatives, quinoline derivatives (for example, quinoline, 3-quinolinecarbonitrile), isoquinoline derivatives, cinnoline derivatives, quinazoline derivatives, quinoxaline derivatives, phthalazine derivatives, purine derivatives, pteridin derivatives, carbazole derivatives, phenanthridine derivatives, acridine derivatives, phenazine derivatives, 1,10-phenanthroline derivatives, adenine derivatives, adenosine derivatives, guanine derivatives, guanosine derivatives, uracil derivatives, and uridine derivatives.

Furthermore, specific examples of the nitrogen-containing compound having a carboxy group include aminobenzoic acid, indolecarboxylic acid, and amino acid derivatives (for example, nicotinic acid, alanine, arginine, aspartic acid, glutamic acid, glycine, histidine, isoleucine, glycylleucine, leucine, methionine, phenylalanine, threonine, lysine, 3-aminopyrazine-2-carboxylic acid, and methoxyalanine). Specific examples of the nitrogen-containing compound having a sulfonyl group include 3-pyridinesulfonic acid and pyridinium p-toluenesulfonate. Specific examples of the nitrogen-containing compound having a hydroxyl group, the nitrogen-containing compound having a hydroxyphenyl group, and the alcoholic nitrogen-containing compound include 2-hydroxypyridine, aminocresol, 2,4-quinolinediol, 3-indolemethanol hydrate, monoethanolamine, diethanolamine, triethanolamine, N-ethyldiethanolamine, N,N-diethylethanolamine, triisopropanolamine, 2,2'-iminodiethanol, 2-aminoethanol, 3-amino-1-propanol, 4-amino-1-butanol, 4-(2-hydroxyethyl)morpholine, 2-(2-hydroxyethyl)pyridine, 1-(2-hydroxyethyl)piperazine, 1-[2-(2-hydroxyethoxy)ethyl]piperazine, piperidine ethanol, 1-(2-hydroxyethyl)pyrrolidine, 1-(2-hydroxyethyl)-2-pyrrolidone, 3-piperidino-1,2-propanediol, 3-pyrrolidino-1,2-propanediol, 8-hydroxyjulolidine, 3-quinuclidinol, 3-tropanol, 1-methyl-2-pyrrolidine ethanol, 1-aziridine ethanol, N-(2-hydroxyethyl)phthalimide, and N-(2-hydroxyethyl)isonicotinamide. Specific examples of the amide derivative include formamide, N-methylformamide, N,N-dimethylformamide, acetamide, N-methylacetamide, N,N-dimethylacetamide, propionamide, and benzamide. Specific examples of the imide derivative include phthalimide, succinimide, and maleimide.

The content of the basic compound in the composition for forming an underlayer film for lithography of the present embodiment is not particularly limited, but is preferably 0.001 to 2 parts by mass, more preferably 0.01 to 1 parts by mass, further preferably 0.05 to 0.5 parts by mass based on 100 parts by mass of the material for forming an underlayer film for lithography. The content is set within the above range to result in a tendency to improve preservation stability without excessively interrupting a crosslinking reaction.

### [Other component(s)]

In addition, the composition for forming an underlayer film for lithography of the present embodiment may contain other resins and/or compounds for the purpose of imparting heat curability and controlling absorbance. Such other resins and/or compounds include naphthol resins, xylene resins, naphthol-modified resins, phenol-modified resins of naphthalene resins, polyhydroxystyrene, dicyclopentadiene resins, (meth)acrylate, dimethacrylate, trimethacrylate, tetramethacrylate, resins having a naphthalene ring such as vinylnaphthalene and polyacenaphthylene, resins having a biphenyl ring such as phenanthrenequinone and fluorene, resins having a heterocyclic ring having a hetero atom such as thiophene and indene, and resins not containing an aromatic ring; rosin-based resins, and resins or compounds including an alicyclic structure, such as cyclodextrin, adamantane(poly)ol, tricyclodecane(poly)ol and derivatives thereof, but are not particularly limited thereto. Furthermore, the composition for forming an underlayer film for lithography of the present embodiment can also contain an additive known in the art such as an ultraviolet absorber, a surfactant, a colorant and a nonionic surfactant.

### [Underlayer film for lithography, and multilayer resist pattern forming method]

An underlayer film for lithography of the present embodiment is formed by using the composition for forming an underlayer film for lithography described above.

In addition, a multilayer resist pattern forming method of the present embodiment can include forming an underlayer film on a substrate by using the composition for forming an underlayer film for lithography described above, forming at least one photoresist layer on the underlayer film, thereafter irradiating a required region of the photoresist layer with radiation, and developing it.

Furthermore, a multilayer resist pattern forming method of the present embodiment can include forming an underlayer film on a substrate by using the material for forming an underlayer film for lithography described above, forming an intermediate layer film on the underlayer film by using a silicon atom-containing resist intermediate layer film material, forming at least one photoresist layer on the intermediate layer film, thereafter irradiating a required region of the photoresist layer with radiation, developing it to form a resist pattern, thereafter etching the intermediate layer film with the resist pattern as a mask, etching the underlayer film with the obtained intermediate layer film pattern as an etching mask, and etching the substrate with the obtained underlayer film pattern as an etching mask, to form a pattern on the substrate.

The underlayer film for lithography of the present embodiment is not particularly limited in terms of the forming method thereof as long as it is formed from the composition for forming an underlayer film for lithography described above, and a method known in the art can be applied. For example, the underlayer film can be formed by applying the composition for forming an underlayer film for lithography described above on the substrate by a known coating method or printing method such as spin coating or screen printing, and thereafter removing an organic solvent by volatilization or the like. The underlayer film is desirably baked upon forming in order to suppress the occurrence of the mixing phenomenon with an upperlayer resist and also promote a crosslinking reaction. In this case, the baking temperature is not particularly limited, but it is preferably within the range of 80 to 450°C, and more preferably 200 to 400°C. In addition, the baking time is not also particularly limited, but is preferably within the range of 10 to 300 seconds. Herein, the thickness of the underlayer film can be appropriately selected depending on the required properties, and is not particularly limited, but the thickness is usually preferably about 30 to 20,000 nm, more preferably 50 to 15,000 nm. After the underlayer film is prepared, in the case of a two-layer process, a silicon-containing resist layer or a usual single-layer resist including a hydrocarbon is prepared on the underlayer film, and in the case of a three-layer process, a silicon-containing intermediate layer is prepared on the underlayer film, and a single-layer resist layer not containing silicon is prepared on the silicon-containing intermediate layer. In these cases, a photoresist material for forming the resist layer, which can be used, is a known one.

After the underlayer film is prepared on the substrate, in the case of a two-layer process, a silicon-containing resist layer or a usual single-layer resist including a hydrocarbon can be prepared on the underlayer film, and in the case of a three-layer process, a silicon-containing intermediate layer can be prepared on the underlayer film, and a single-layer resist layer not containing silicon can be prepared on the silicon-containing intermediate layer. In these cases, a photoresist material for forming the resist layer, which can be used, is appropriately selected from known ones, and is not particularly limited.

As the silicon-containing resist material for a two-layer process, a positive-type photoresist material is preferably used, which contains a silicon atom-containing polymer such as a polysilsesquioxane derivative or a vinylsilane derivative used as a base polymer in the viewpoint of oxygen gas-etching resistance, and an organic solvent, an acid generator and if necessary a basic compound. Herein, as the silicon atom-containing polymer, a known polymer used in such a resist material can be used.

As the silicon-containing intermediate layer for a three-layer process, a polysilsesquioxane-based intermediate layer is preferably used. The intermediate layer is allowed to have an effect as an antireflective film, and thus can allow reflection to be suppressed. For example, if a material including many aromatic groups and having a high substrate-etching resistance is used for the underlayer film in a 193 nm exposure process, a k-value tends to be increased to increase substrate reflection, but the reflection can be suppressed by the intermediate layer to thereby make the substrate reflection 0.5% or less. For the intermediate layer having such an antireflection effect, polysilsesquioxane into which a phenyl group or a light-absorbing group having a silicon-silicon bond for 193 nm exposure is introduced and which is to be crosslinked with an acid or heat is preferably used.

An intermediate layer formed by the Chemical Vapour Deposition (CVD) method can also be used. As the intermediate layer having a high effect as an antireflective film, prepared by the CVD method, for example, a SiON film is known. In general, the intermediate layer is formed by a wet process such as a spin coating method or screen printing rather than the CVD method in terms of simplicity and cost effectiveness. Herein, the upperlayer resist in a three-layer process may be of positive-type or negative-type, and the same one as a commonly used single-layer resist can be used therefor.

Furthermore, the underlayer film of the present embodiment can also be used as a usual antireflective film for use in a single-layer resist or a usual underlying material for suppressing pattern collapse. The underlayer film of the present embodiment can also be expected to serve as a hard mask for underlying processing because of being excellent in etching resistance for underlying processing.

In the case where a resist layer is formed by the photoresist material, a wet process such as a spin coating method or screen printing is preferably used as in the case of forming the underlayer film. The resist material is coated by a spin coating method or the like and then usually pre-baked, and such pre-baking is preferably performed in the range of 80 to 180°C for 10 to 300 seconds. Thereafter, in accordance with an ordinary method, the resultant can be subjected to exposure, post-exposure bake (PEB), and development to obtain a resist pattern. Herein, the thickness of the resist film is not particularly limited, but generally, it is preferably 30 to 500 nm and more preferably 50 to 400 nm.

Light for use in exposure may be appropriately selected depending on the photoresist material to be used. In general, examples thereof include high energy radiation having a wavelength of 300 nm or less, specifically, excimer lasers of 248 nm, 193 nm, and 157 nm, a soft X-ray of 3 to 20 nm, electron beam, and an X-ray.

The resist pattern formed by the above method is a pattern whose collapse is suppressed by the underlayer film of the present embodiment. Therefore, the underlayer film of the present embodiment can be used to thereby obtain a finer pattern, and an exposure amount necessary for obtaining such a resist pattern can be reduced.

Then, the obtained resist pattern is used as a mask to perform etching. As the etching of the underlayer film in a two-layer process, gas etching is preferably used. As the gas etching, etching using oxygen gas is suitable. In addition to oxygen gas, an inert gas such as He and Ar, and CO, CO₂, NH₃, SO₂, N₂, NO₂, and H₂ gases can also be added. The gas etching can also be performed not using oxygen gas but using only CO, CO₂, NH₃, N₂, NO₂, and H₂ gases. In particular, the latter gases are used for protecting a side wall for preventing a pattern side wall from being undercut. On the other hand, also in the etching of the intermediate layer in a three-layer process, gas etching is preferably used. As the gas etching, the same one as the one described in a two-layer process can be applied. In particular, the intermediate layer is preferably processed in a three-layer process using a fluorocarbon gas with the resist pattern as a mask. Thereafter, as described above, the intermediate layer pattern is used as a mask to perform, for example, oxygen gas etching, thereby processing the underlayer film.

Herein, in the case where an inorganic hard mask intermediate layer film is formed as the intermediate layer, a silicon oxide film, a silicon nitride film, and a silicon oxynitride film (SiON film) are formed by the CVD method, the ALD method, and the like. The nitride film forming method is described in, for example, Japanese Patent Laid-Open No. 2002-334869 (Patent Literature 6) and WO 2004/066377 (Patent Literature 7).

While the photoresist film can be directly formed on such an intermediate layer film, an organic antireflective film (BARC) may also be formed on the intermediate layer film by spin coating, and the photoresist film may also be formed thereon.

As the intermediate layer, a polysilsesquioxane-based intermediate layer is also preferably used. The resist intermediate layer film is allowed to have an effect as an antireflective film, and thus can allow reflection to be suppressed. A specific material for the polysilsesquioxane-based intermediate layer is described in, for example, Japanese Patent Laid-Open No. 2007-226170 (Patent Literature 8 described above) and Japanese Patent Laid-Open No. 2007-226204 (Patent Literature 9 described above).

The next etching of the substrate can also be performed by an ordinary method, and, for example, when the substrate is made of SiO₂ or SiN, etching with mainly a fluorocarbon gas can be performed, and when the substrate is made of p-Si, Al or W, etching mainly using a chlorine-based gas or bromine-based gas can be performed. In the case where processing of the substrate is performed by the etching with a fluorocarbon gas, the silicon-containing resist in a two-layer resist process and the silicon-containing intermediate layer in a three-layer process are peeled off at the same time as the processing of the substrate. On the other hand, in the case where the substrate is processed by the etching with a chlorine-based gas or bromine-based gas, the silicon-containing resist layer or the silicon-containing intermediate layer is peeled off separately, and is generally peeled off by dry etching with a fluorocarbon gas after the substrate is processed.

The underlayer film of the present embodiment is excellent in etching resistance of such a substrate.

Herein, the substrate that can be used is appropriately selected from ones known in the art, and is not particularly limited, but includes Si, α-Si, p-Si, SiO₂, SiN, SiON, W, TiN and Al substrates. In addition, the substrate may also be a laminate having a processed film (processed substrate) on a base material (support). Such a processed film includes various Low-k films made of Si, SiO₂, SiON, SiN, p-Si, α-Si, W, W-Si, Al, Cu, and Al-Si, and stopper films thereof, and a material different from the base material (support) is usually used therefor. Herein, the thickness of the substrate to be processed or the processed film is not particularly limited, but it is usually preferably about 50 to 10,000 nm and more preferably 75 to 5,000 nm.

### [Purification method of compound or resin]

A purification method of the compound or the resin of the present embodiment includes a step of bringing a solution (hereinafter, sometimes referred to as "solution (A)") including an organic solvent optionally immiscible with water, and the compound represented by the formula (1) or the resin obtained with the compound as a monomer into contact with an acidic aqueous solution for extraction. The purification method of the present embodiment can reduce the content of various metals that can be included as impurities in the compound or the resin.

More specifically, in the present embodiment, an extraction treatment can be performed by dissolving the compound or the resin in the organic solvent optionally immiscible with water, and further bringing the solution into contact with an acidic aqueous solution. Thus, a metal content included in the solution (A) including the compound or the resin can be transferred to an aqueous phase, and an organic phase and the aqueous phase can be then separated to thereby provide the compound or the resin in which the metal content is reduced.

The compound or the resin to be used in the present embodiment may be a single form, but can also be a mixture of two or more. In addition, the compound or the resin may also contain various surfactants, various crosslinking agents, various acid generators, various stabilizers, and the like.

The organic solvent optionally immiscible with water, to be used in the present invention, is not particularly limited, but is preferably an organic solvent which can be safely applied to a semiconductor manufacturing process. The amount of the organic solvent to be used is usually about 1 to 100 times by mass the amount of the compound or the resin.

Specific examples of the solvent to be used include ethers such as diethyl ether and diisopropyl ether, esters such as ethyl acetate, n-butyl acetate and isoamyl acetate, ketones such as methyl ethyl ketone, methyl isobutyl ketone, ethyl isobutyl ketone, cyclohexanone, cyclopentanone, 2-heptanone and 2-pentanone, glycol ether acetates such as ethylene glycol monoethyl ether acetate, ethylene glycol monobutyl ether acetate, propylene glycol monomethyl ether acetate (PGMEA) and propylene glycol monoethyl ether acetate, aliphatic hydrocarbons such as n-hexane and n-heptane, aromatic hydrocarbons such as toluene and xylene, and halogenated hydrocarbons such as methylene chloride and chloroform. Among them, toluene, 2-heptanone, cyclohexanone, cyclopentanone, methyl isobutyl ketone, propylene glycol monomethyl ether acetate, ethyl acetate and the like are preferable, methyl isobutyl ketone, ethyl acetate, cyclohexanone and propylene glycol monomethyl ether acetate are more preferable, and methyl isobutyl ketone and ethyl acetate are still more preferable. Methyl isobutyl ketone, ethyl acetate, and the like are relatively high in saturation solubility of the compound of the present embodiment or the resin of the present embodiment and relatively low in the boiling point, and therefore can allow the burden in industrial distilling off of the solvent or in a step of removing the solvent by drying to be reduced.

These solvents can be used singly or as a mixture of two or more thereof.

The acidic aqueous solution to be used in the present invention is appropriately selected from aqueous solutions in which an organic or inorganic compound commonly known is dissolved in water. Examples include an aqueous solution (mineral acid) in which a mineral acid such as hydrochloric acid, sulfuric acid, nitric acid or phosphoric acid is dissolved in water, or an aqueous solution (organic acid) in which an organic acid such as acetic acid, propionic acid, oxalic acid, malonic acid, succinic acid, fumaric acid, maleic acid, tartaric acid, citric acid, methanesulfonic acid, phenolsulfonic acid, p-toluenesulfonic acid or trifluoroacetic acid is dissolved in water. These acidic aqueous solutions can be used singly or in combinations of two or more thereof. Among these acidic aqueous solutions, an aqueous solution of sulfuric acid, nitric acid, and carboxylic acid such as acetic acid, oxalic acid, tartaric acid and citric acid is preferable, an aqueous solution of sulfuric acid, oxalic acid, tartaric acid and citric acid is further preferable, and an aqueous solution of oxalic acid is particularly preferable. It is considered that a polyvalent carboxylic acid such as oxalic acid, tartaric acid and citric acid is coordinated with a metal ion to exert a chelating effect, and therefore can allow a metal to be more removed. In addition, the water to be here used is preferably water having a low metal content according to the purpose of the present invention, such as ion-exchange water.

The pH of the acidic aqueous solution to be used in the present invention is not particularly limited, but the aqueous solution, if having a too high acidity, is not preferable because of sometimes having an adverse effect on the compound or the resin. The pH is usually in the range from about 0 to 5, more preferably about 0 to 3.

The amount of the acidic aqueous solution to be used in the present invention is not particularly limited, but the aqueous solution, if used in a too small amount, causes a need for an increase in the number of extractions for metal removal, and on the contrary, the aqueous solution, if used in a too large amount, causes an increase in the total amount of the liquid, thereby sometimes resulting in an operational problem. The amount of the aqueous solution to be used is usually 10 to 200% by mass, preferably 20 to 100% by mass relative to the solution of the compound or the resin dissolved in the organic solvent.

In the purification method of the present invention, the acidic aqueous solution is brought into contact with the solution (A) including the compound or the resin and the organic solvent optionally immiscible with water, to thereby extract the metal content.

In the purification method of the present invention, the solution (A) preferably further includes an organic solvent optionally miscible with water. In the purification method of the present invention, when the solution (A) includes the organic solvent optionally miscible with water, the amount of the compound or the resin to be charged can be increased, and liquid separating property can be enhanced to perform purification at a high pot efficiency. The method for adding the organic solvent optionally miscible with water to the solution (A) is not particularly limited. For example, any of a method in which the organic solvent optionally miscible with water is added to the solution including the organic solvent optionally immiscible with water in advance; a method in which the organic solvent optionally miscible with water is added to water or the acidic aqueous solution in advance; and a method in which the organic solvent optionally miscible with water is added after the solution including the organic solvent optionally immiscible with water is brought into contact with water or the acidic aqueous solution can be adopted, but a method in which the organic solvent optionally miscible with water is added to the solution including the organic solvent optionally immiscible with water in advance is preferable in terms of operation workability and ease of maintenance of the amount to be charged.

The "organic solvent optionally miscible with water" is not particularly limited, but is preferably an organic solvent that is optionally miscible with water and that can be safely applied to a semiconductor manufacturing process. The content of the "organic solvent optionally miscible with water" is not particularly limited as long as a solution phase and an aqueous phase are separated, but the "organic solvent optionally miscible with water" can be usually used in an amount of about 0.1 to 100 times by mass the total amount of the compound represented by the formula (1) used or the resin having the structure represented by the formula (2).

Specific examples of the organic solvent optionally miscible with water include ethers such as tetrahydrofuran and 1,3-dioxolane; alcohols such as methanol, ethanol and isopropanol; ketones such as acetone and N-methylpyrrolidone; glycol ethers such as ethylene glycol monoethyl ether, ethylene glycol monobutyl ether, propylene glycol monomethyl ether (PGME) and propylene glycol monoethyl ether, and aliphatic hydrocarbons. Among them, N-methylpyrrolidone, propylene glycol monomethyl ether, and the like are preferable, and particularly N-methylpyrrolidone and propylene glycol monomethyl ether are preferable. These solvents can be used singly or as a mixture of two or more thereof.

In the present embodiment, the temperature in bringing of the solution (A) into contact with the acidic aqueous solution, namely, in performing of an extraction treatment is usually in the range from 20 to 90°C, preferably 30 to 80°C. The extraction operation is not particularly limited, but is performed by, for example, well mixing with stirring or the like and thereafter standing. Thus, the metal content included in the solution including the compound or the resin and the organic solvent is transferred to the aqueous phase. In addition, the operation can allow the acidity of the solution to be reduced, suppressing the deterioration of properties of the compound or the resin.

The mixed solution is separated to the solution phase including the compound or the resin and the organic solvent, and the aqueous phase by standing, and therefore the solution including the compound or the resin and the organic solvent is recovered by decantation or the like. The standing time is not particularly limited, but the standing time is preferably adjusted from the viewpoint of providing better separation to the solution phase including the organic solvent, and the aqueous phase. The standing time is usually 1 minute or more, preferably 10 minutes or more, more preferably 30 minutes or more. In addition, the extraction treatment may be performed only once, but is also effectively performed with operations such as mixing, standing and separation being repeatedly performed multiple times.

In the present embodiment, after the extraction treatment by the step of bringing the solution (A) into contact with the acidic aqueous solution is performed, a step of further performing an extraction treatment with water is preferably performed. That is, after the extraction treatment with the acidic aqueous solution is performed, the solution including the compound or the resin and the organic solvent, extracted and recovered from the aqueous solution, is preferably further subjected to an extraction treatment with water. The extraction treatment with water is not particularly limited, but can be performed by, for example, well mixing with stirring or the like and thereafter standing. The solution obtained after the standing is separated to the solution phase including the compound or the resin and the organic solvent, and the aqueous phase, and therefore the solution phase including the compound or the resin and the organic solvent can be recovered by decantation or the like.

In addition, the water to be here used is preferably water having a low metal content according to the purpose of the present embodiment, such as ion-exchange water. The extraction treatment may be performed only once, but is also effectively performed with operations such as mixing, standing and separation being repeatedly performed multiple times. In addition, conditions in the extraction treatment, such as the ratio of both to be used, the temperature and the time, are not particularly limited, but may be the same as in the case of the contact treatment with the acidic aqueous solution above.

The water content that can be incorporated in the solution thus obtained, including the compound or the resin and the organic solvent, can be easily removed by performing an operation such as distillation under reduced pressure. In addition, an organic solvent can be if necessary added to adjust the concentration of the compound or the resin to any concentration.

The method of isolating the compound or the resin from the resulting solution including the compound or the resin and the organic solvent is not particularly limited, and can be performed by a known method such as removal under reduced pressure, separation by reprecipitation and a combination thereof. If necessary, a known treatment such as a concentration operation, a filtration operation, a centrifugation operation and a drying operation can be performed.

### Examples

Hereinafter, the present invention will be described by Synthesis Examples and Examples in more detail, but the present invention is not limited thereto at all.

### (Measurement of carbon concentration and oxygen concentration)

The carbon concentration and the oxygen concentration (% by mass) were measured by organic element analysis.

### Apparatus: CHN CORDER MT-6 (manufactured by Yanaco Bunseki Kogyo Co.)

### (Molecular Weight)

Measurement was performed by LC-MS analysis using Acquity UPLC/MALDI-Synapt HDMS manufactured by Water.

### (Molecular Weight Measurement)

Gel permeation chromatography (GPC) analysis was used to determine the weight average molecular weight (Mw) and the number average molecular weight (Mn) in terms of polystyrene, and to determine the degree of dispersion (Mw/Mn).
Apparatus: Shodex GPC-101 type (manufactured by Showa Denko K. K.)
Column: KF-80M × 3
Eluent: THF 1 mL/min
Temperature: 40°C

### (Evaluation of heat resistance)

An EXSTAR 6000 DSC apparatus manufactured by SII NanoTechnology Inc. was used, and about 5 mg of a sample was placed in an unsealed aluminum container and heated to 500°C at a rate of temperature rise of 10°C/min in a nitrogen gas (30 mL/min) stream. In this time, a temperature at which a reducing portion appeared on the base line was defined as a pyrolysis temperature (Tg), and the heat resistance was evaluated according to the following criteria.
Evaluation A: pyrolysis temperature ≥ 150°C
Evaluation C: pyrolysis temperature < 150°C

### (Evaluation of solvent solubility)

Each compound was dissolved at 23°C so as to be provided as a 5% by mass solution in propylene glycol monomethyl ether (PGME), thereafter the solution was left to stand at 5°C for 30 days, and the results were evaluated according to the following criteria.
Evaluation A: no precipitate was visually confirmed
Evaluation C: any precipitate was visually confirmed

### (Production Example 1) Synthesis of BisF-1

A container having an inner volume of 200 mL, equipped with a stirrer, a condenser and a burette, was prepared. To the container were charged 30 g (161 mmol) of 4,4'-biphenol (reagent produced by Tokyo Chemical Industry Co., Ltd.), 15 g (82 mmol) of 4-biphenylaldehyde (produced by Mitsubishi Gas Chemical Co., Ltd.) and 100 mL of butyl acetate, and 3.9 g (21 mmol) of p-toluenesulfonic acid (reagent produced by Kanto Chemical Co., Inc.) was added thereto to prepare a reaction liquid. The reaction liquid was stirred at 90°C for 3 hours to perform a reaction. Then, the reaction liquid was concentrated, 50 g of heptane was added to precipitate a reaction product, and the resultant was cooled to room temperature followed by filtration for separation. A solid obtained by filtration was dried, and thereafter separated and purified by column chromatography to thereby provide 5.8 g of an objective compound (BisF-1) represented by the following formula.

Herein, the following peaks were observed by 400 MHz-¹H-NMR, and it was confirmed that the compound had a chemical structure of the following formula.
¹H-NMR: (d-DMSO, internal standard TMS)
δ (ppm) 9.4 (4H, O-H), 6.8-7.8 (22H, Ph-H), 6.2 (1H, C-H)

The molecular weight of the resulting compound was measured by the above method, and as a result, it was 536.

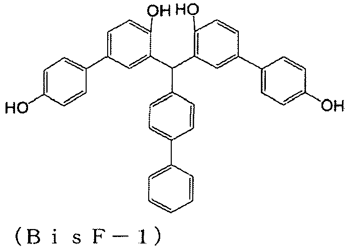

### (Production Example 2) Synthesis of BisF-2

Except that 4,4'-biphenol was changed to 2,2'-biphenol, the same manner as in Production Example 1 was performed to provide 3.0 g of an objective mixture (BisF-2) represented by the following formula.

Herein, the following peaks were observed by 400 MHz-¹H-NMR, and it was confirmed that the compound had a chemical structure of the following formula.
¹H-NMR: (d-DMSO, internal standard TMS)
δ (ppm) 9.3 (4H, O-H), 6.8-7.8 (22H, Ph-H), 6.3 (1H, C-H)

The molecular weight of the resulting compound was measured by the above method, and as a result, it was 536.

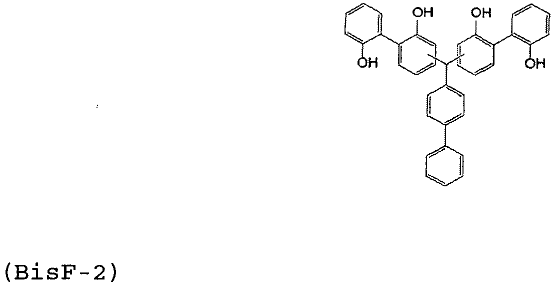

### (Synthesis Example 1) Synthesis of BisF-1-CH4

To a container having an inner volume of 1000 mL, equipped with a stirrer, a condenser and a burette, were charged 6.7 g (12.5 mmol) of BisF-1 obtained above, 108 g (810 mmol) of potassium carbonate and 200 mL of dimethylformamide, 250 g (1.53 mol) of bromocyclohexane was added thereto, and the reaction liquid was stirred at 110°C for 24 hours to perform a reaction. Then, the reaction liquid was concentrated, 500 g of pure water was added to precipitate a reaction product, and the resultant was cooled to room temperature followed by filtration for separation. A solid obtained was subjected to filtration and dried, and thereafter separated and purified by column chromatography to thereby provide 5.0 g of an objective compound (BisF-1-CH4) represented by the following formula.

The resulting compounds were subjected to NMR measurement in the measurement conditions, and thus the following peaks were observed and it was confirmed that the compounds had respective chemical structures of the following formulae.
¹H-NMR: (d-DMSO, internal standard TMS)
δ (ppm) 6.8-7.8 (22H, Ph-H), 6.2 (1H, C-H), 1.4-4.5 (44H, Cy-H)/Cy-H

The molecular weight of BisF-1-CH4 obtained was 865. In addition, the carbon concentration was 84.7% by mass and the oxygen concentration was 7.4% by mass.

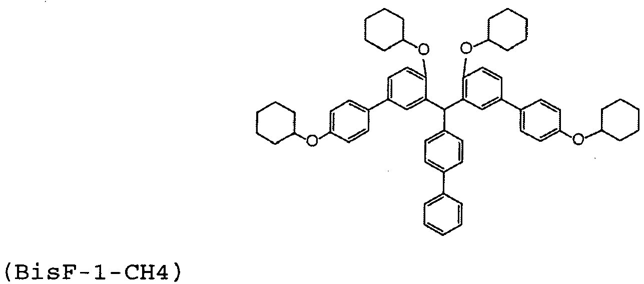

### (Synthesis Example 2) Synthesis of BisF-1-CH2

Except that the amount of potassium carbonate was changed to 54 g (405 mmol) and the amount of bromocyclohexane was changed to 125 g (0.77 mol), the same manner as in Synthesis Example 1 was performed to provide 2.0 g of an objective mixture (BisF-1-CH2) represented by the following formula.

The resulting compound was subjected to NMR measurement in the measurement conditions, and thus the following peaks were observed and it was confirmed that the compound had a chemical structure of the following formula.
¹H-NMR: (d-DMSO, internal standard TMS)
   δ (ppm) 9.4 (2H, O-H), 6.8-7.8 (22H, Ph-H), 6.2 (1H, C-H), 1.4-4.5 (22H, Cy-H)/Cy-H

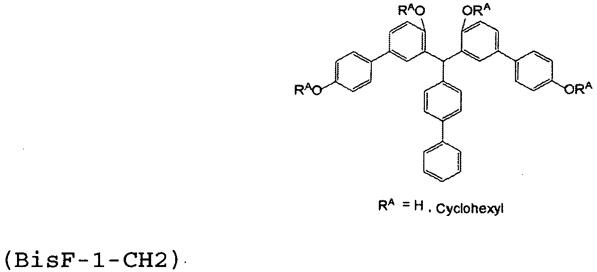

### (Synthesis Example 3) Synthesis of BisF-1-ME4

Except that bromocyclohexane was changed to 217 g (1.53 mol) of methyl iodide, the same manner as in Synthesis Example 1 was performed to provide 2.0 g of an objective compound (BisF-1-ME4) represented by the following formula.

The resulting compound was subjected to NMR measurement in the measurement conditions, and thus the following peaks were observed and it was confirmed that the compound had a chemical structure of the following formula.
¹H-NMR: (d-DMSO, internal standard TMS)
   δ (ppm) 6.8-7.8 (22H, Ph-H), 6.2 (1H, C-H), 3.8 (12H, CH3)

The molecular weight of BisF-2-ME4 obtained was 592. In addition, the carbon concentration was 83.1% by mass and the oxygen concentration was 10.8% by mass.

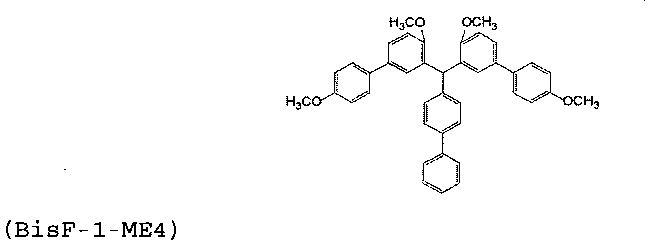

### (Synthesis Example 4) Synthesis of BisF-1-PH4

To a container having an inner volume of 1000 mL, equipped with a stirrer, a condenser and a burette, were charged 3.4 g (6.3 mmol) of BisF-1 obtained above, 26 g (80 mmol) of cesium carbonate, 0.8 g (4 mmol) of copper iodide, 1.7 g (12 mmol) of dimethyl glycine hydrochloride and 80 mL of dioxane, 8.2 g (40 mmol) of iodobenzene was added thereto, and the reaction liquid was stirred at 90°C for 96 hours to perform a reaction. Then, 500 mL of ethyl acetate was added to precipitate a reaction product, and the resultant was cooled to room temperature followed by filtration for separation. The resulting solid was filtered and dried, and thereafter separated and purified by column chromatography to thereby provide 1.9 g of an objective compound (BisF-1-PH4) represented by the following formula.

The resulting compound was subjected to NMR measurement in the measurement conditions, and thus the following peaks were observed and it was confirmed that the compound had a chemical structure of the following formula.
¹H-NMR: (d-DMSO, internal standard TMS)
   δ (ppm) 6.8-7.8 (42H, Ph-H), 6.2 (1H, C-H)

The molecular weight of BisF-1-PH4 obtained was 840. In addition, the carbon concentration was 87.1% by mass and the oxygen concentration was 7.6% by mass.

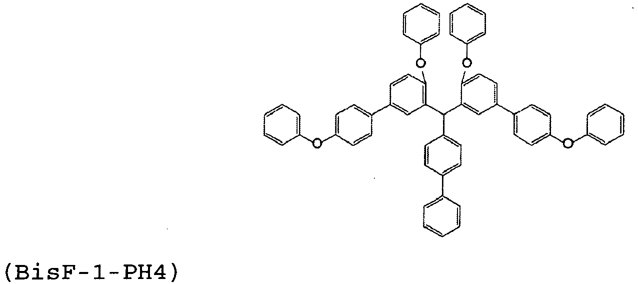

### (Synthesis Example 5) Synthesis of BisF-2-CH4

Except that BisF-1 was changed to BisF-2, the same manner as in Synthesis Example 1 was performed to provide 2.0 g of an objective compound (BisF-2-CH4) represented by the following formula.

The resulting compound was subjected to NMR measurement in the measurement conditions, and thus the following peaks were observed and it was confirmed that the compound had a chemical structure of the following formula.
¹H-NMR: (d-DMSO, internal standard TMS)
   δ (ppm) 6.8-7.8 (22H, Ph-H), 6.3 (1H, C-H), 1.4-4.5 (44H, Cy-H)/Cy-H

The molecular weight of BisF-2-CH4 obtained was 865. In addition, the carbon concentration was 84.7% by mass and the oxygen concentration was 7.4% by mass.

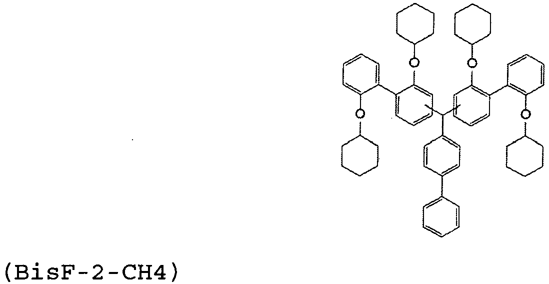

### (Production Example 3)

A four-neck flask having a bottom outlet and an inner volume of 10 L, equipped with a Dimroth condenser, a thermometer and a stirring blade was prepared. To the four-neck flask were charged 1.09 kg (7 mol, produced by Mitsubishi Gas Chemical Company, Inc.) of 1,5-dimethylnaphthalene, 2.1 kg (28 mol as formaldehyde, produced by Mitsubishi Gas Chemical Company, Inc.) of a 40% by mass aqueous formalin solution and 0.97 mL of 98% by mass sulfuric acid (produced by Kanto Chemical Co., Inc.) under a nitrogen stream, and allowed the reaction to run under ordinary pressure for 7 hours with refluxing at 100°C. Thereafter, ethylbenzene (special grade chemical, produced by Wako Pure Chemical Industries, Ltd.) (1.8 kg) as a dilution solvent was added to the reaction solution and left to stand, and then an aqueous phase being a bottom phase was removed. Furthermore, the resultant was neutralized and washed with water, and ethylbenzene and the unreacted 1,5-dimethylnaphthalene were distilled off under reduced pressure, thereby providing 1.25 kg of a dimethylnaphthalene formaldehyde resin as a light-brown solid.

With respect to the molecular weight of the resulting dimethylnaphthalene formaldehyde resin, Mn was 562, Mw was 1168 and Mw/Mn was 2.08. In addition, the carbon concentration was 84.2% by mass, and the oxygen concentration was 8.3% by mass.

Subsequently, a four-neck flask having an inner volume of 0.5 L, equipped with a Dimroth condenser, a thermometer and a stirring blade, was prepared. To the four-neck flask were charged 100 g (0.51 mol) of the dimethylnaphthalene formaldehyde resin obtained as above and 0.05 g of para-toluenesulfonic acid under a nitrogen stream, and heated for 2 hours with the temperature being raised to 190°C, and then stirred. Thereafter, 52.0 g (0.36 mol) of 1-naphthol was further added thereto, and further heated to 220°C to allow the reaction to run for 2 hours. After being diluted with a solvent, the resultant was neutralized and washed with water, and the solvent was removed under reduced pressure to thereby provide 126.1 g of a modified resin (CR-1) as a blackish brown solid.

With respect to the resulting resin (CR-1), Mn was 885, Mw was 2220 and Mw/Mn was 4.17. In addition, the carbon concentration was 89.1% by mass and the oxygen concentration was 4.5% by mass.

### (Examples 1 to 5 and Comparative Examples 1 and 2)

BisF-1-CH4, BisF-1-CH2, BisF-1-ME4, BisF-1-PH4, BisF-2-CH4, BisF-1 and CR-1 above were used to perform evaluation of heat resistance and evaluation of solvent solubility. The results are shown in Table 1.

In addition, each composition for forming an underlayer film for lithography, having composition shown in Table 1, was prepared. Then, a silicon substrate was spin-coated with such a composition for forming an underlayer film, thereafter the resultant was baked at 240°C for 60 seconds and further at 400°C for 120 seconds to prepare each underlayer film having a thickness of 200 nm. The following acid generator, crosslinking agent and organic solvent were used.

Acid generator: di-tert-butyldiphenyliodonium nonafluoromethanesulfonate (DTDPI) produced by Midori Kagaku Co., Ltd.
Crosslinking agent: Nikalac MX270 (Nikalac) produced by Sanwa Chemical Co., Ltd.
Organic solvent: propylene glycol monomethyl ether acetate (PGMEA)
Novolac: PSM4357 produced by Gunei Chemical Industry Co., Ltd.

Then, an etching test was performed under conditions shown below to evaluate etching resistance. The evaluation results are shown in Table 1.

### [Etching test]

Etching apparatus: RIE-10NR manufactured by Samco Inc.
Output: 50 W
Pressure: 20 Pa
Time: 2 min
Etching gas
Ar gas flow rate : CF₄ gas flow rate : O₂ gas flow rate = 50 : 5 : 5 (sccm)

### [Evaluation of Etching Resistance]

The evaluation of etching resistance was performed according to the following procedure.

First, an underlayer film of novolac was prepared under the same conditions as those in Example 1 except that novolac (PSM4357 produced by Gunei Chemical Industry Co., Ltd.) was used instead of the compound (BisF-1-CH4) used in Example 1. Then, the etching test was performed with respect to the underlayer film of novolac as a subject, and the etching rate in that time was measured.

Then, the etching test was performed with respect to the underlayer film of each of Examples 1 to 5 and Comparative Examples 1 to 2 as a subject, and the etching rate in that time was measured.

Then, the etching resistances were evaluated according to the following criteria based on the etching rate of the underlayer film of novolac.

### [Evaluation Criteria]

A: etching rate of less than -10% compared with the underlayer film of novolac
B: etching rate of -10% to +5% compared with underlayer film of novolac
C: etching rate of more than +5% compared with the underlayer film of novolac

Then, a SiO₂ substrate having a thickness of 300 nm was coated with each of the compositions for forming an underlayer film for lithography, including BisF-1-CH4, BisF-1-CH2, BisF-1-ME4, BisF-1-PH4, BisF-2-CH4, BisF-1 and CR-1, respectively, and the resultant was baked at 240°C for 60 seconds and further at 400°C for 120 seconds to thereby form each underlayer film having a thickness of 70 nm. A resist solution for ArF was coated on the underlayer film, and baked at 130°C for 60 seconds to thereby form a photoresist layer having a film thickness of 140 nm. Herein, as the resist solution for ArF, one prepared by blending 5 parts by mass of the compound of the following formula (11), 1 part by mass of triphenylsulfonium nonafluoromethanesulfonate, 2 parts by mass of tributylamine, and 92 parts by mass of PGMEA was used.

A compound of formula (11) was formed into a reaction solution by dissolving 4.15 g of 2-methyl-2-methacryloyloxyadamantane, 3.00 g of methacryloyloxy-γ-butyrolactone, 2.08 g of 3-hydroxy-1-adamantyl methacrylate and 0.38 g of azobisisobutyronitrile in 80 mL of tetrahydrofuran. The reaction solution was subjected to polymerization under a nitrogen atmosphere for 22 hours with the reaction temperature being kept at 63°C, and thereafter the reaction solution was dropped in 400 mL of n-hexane. The product resin thus obtained was solidified and purified, and a white powder produced was collected by filtration and dried under reduced pressure at 40°C overnight.

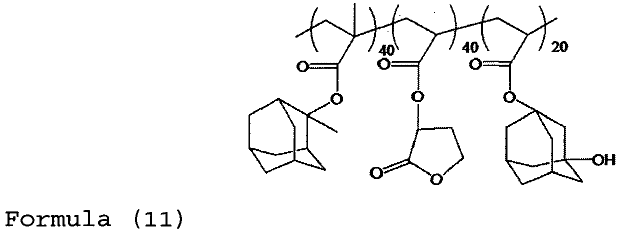

In the formula (11), the numerals 40, 40, and 20 indicate the proportions of the respective constituent units, and do not mean a block copolymer.

Then, the photoresist layer was exposed by using an electron beam lithography apparatus (ELS-7500, produced by Elionix, Inc., 50 keV), baked at 115°C for 90 seconds (PEB), and developed with a 2.38% by mass aqueous tetramethylammonium hydroxide (TMAH) solution for 60 seconds, thereby providing a positive-type resist pattern.

The shape of the resist pattern obtained in each of Examples and Comparative Examples was observed by using an electron microscope (S-4800) manufactured by Hitachi Ltd. The shape of the resist pattern after development was evaluated as follows: any shape having no pattern collapse observed and having good rectangularity was rated as "Good" and any shape other than those rated as "Good" was rated as "Not good". In addition, as a result of observation, the minimum line width where no pattern collapse was observed and rectangularity was good was defined as the resolution and used as an evaluation index. Furthermore, the minimum amount of electronic beam energy, where a good pattern shape could be drawn, was defined as the sensitivity and used as an evaluation index. The results are shown in Table 1.

### (Comparative Example 3)

Except that formation of an underlayer film was not performed, the same manner as in Example 1 was preformed to directly form a photoresist layer on a SiO₂ substrate, thereby providing a positive-type resist pattern, and the same evaluation was performed. The results are shown in Table 1.

[Table 1]

**Table 1**

| | Material for forming underlayer film | Evaluation of heat resistance | Evaluation of solvent solubility | Component ratio of composition for forming underlayer film | | | | Evaluation of etching resistance | Evaluation of resist performance | | |
|---|---|---|---|---|---|---|---|---|---|---|---|
| | | | | Material for forming underlayer film (parts by mass) | Solvent (parts by mass) | Acid generator (parts by mass) | Crosslinking agent (parts by mass) | | Resolution (nmL/S) | Sensitivity (µC/cm²) | Resist pattern shape after development |
| Example 1 | BisF-1-CH4 | A | A | 10 | 90 | 0.5 | 0.5 | A | 50 | 12 | Good |
| Example 2 | BisF-1-CH2 | A | A | 10 | 90 | 0.5 | 0.5 | A | 50 | 12 | Good |
| Example 3 | BisF-1-ME4 | A | A | 10 | 90 | 0.5 | 0.5 | A | 50 | 12 | Good |
| Example 4 | BisF-1-PH4 | A | A | 10 | 90 | 0.5 | 0.5 | A | 50 | 12 | Good |
| Example 5 | BisF-2-CH4 | A | A | 10 | 90 | 0.5 | 0.5 | A | 50 | 12 | Good |
| Comparative Example 1 | BisF-1 | A | A | 10 | 90 | 0.5 | 0.5 | B | 55 | 12 | Not good |
| Comparative Example 2 | CR-1 | C | C | 10 | 90 | 0.5 | 0.5 | C | - | - | - |
| Comparative Example 3 | | - | - | No underlayer film formed | | | | | 80 | 26 | Not good |

As can be seen from Table 1, it was confirmed that all of heat resistance, solvent solubility and etching resistance were good in Example 1 in which BisF-1-CH4 being the compound of the present invention was used. On the other hand, heat resistance and etching resistance were good, but solvent solubility was not good in Comparative Example 1 in which the polyphenol compound BisF-1 was used. In addition, all of heat resistance, solvent solubility and etching resistance were not good in Comparative Example 2 in which CR-1 (phenol-modified dimethylnaphthalene formaldehyde resin) was used, and evaluation of resist performance was not performed.

In addition, it was confirmed that the resist pattern shape after development was good and no defects were observed in Example 1. On the other hand, it was confirmed that the resist pattern shape after development was not good and many defects were also observed in Comparative Example 1. The reason was presumed as follows: BisF-1 used in Comparative Example 1 had a low solubility in the coating solvent.

Furthermore, it was confirmed that resolution and sensitivity were significantly excellent in Example 1 as compared with Comparative Example 3 in which underlayer film formation was omitted.

It was indicated from the difference in resist pattern shape after development that the material for forming an underlayer film for lithography in Example 1 was good in adhesiveness with a resist material.

### (Example 6)

Then, the composition for forming an underlayer film for lithography used in Example 1 was coated on a SiO₂ substrate having a film thickness of 300 nm, and baked at 240°C for 60 seconds and further at 400°C for 120 seconds to thereby form an underlayer film having a film thickness of 80 nm. A silicon-containing intermediate layer material was coated on the underlayer film, and baked at 200°C for 60 seconds to thereby form an intermediate layer film having a film thickness of 35 nm. Furthermore, the resist solution for ArF was coated on the intermediate layer film, and baked at 130°C for 60 seconds to thereby form a photoresist layer having a film thickness of 150 nm. Herein, as the silicon-containing intermediate layer material, the silicon atom-containing polymer described in <Synthesis Example 1> in Japanese Patent Laid-Open No. 2007-226170 was used.

Then, the photoresist layer was exposed with a mask by using an electron beam lithography apparatus (ELS-7500, produced by Elionix, Inc., 50 keV), baked at 115°C for 90 seconds (PEB), and developed with a 2.38% by mass aqueous tetramethylammonium hydroxide (TMAH) solution for 60 seconds, thereby providing a positive-type resist pattern of 55 nm L/S (1:1).

Thereafter, dry etching processing of a silicon-containing intermediate layer film (SOG) was performed by using RIE-10NR manufactured by Samco Inc. with the obtained resist pattern as a mask, and subsequently, dry etching processing of an underlayer film and dry etching processing of a SiO₂ film were sequentially performed with the obtained silicon-containing intermediate layer film pattern as a mask and the obtained underlayer film pattern as a mask, respectively.

Respective etching conditions are as shown below.

### (Etching conditions of resist intermediate layer film with resist pattern)

Output: 50 W
Pressure: 20 Pa
Time: 1 min
Etching gas
Ar gas flow rate : CF₄ gas flow rate : O₂ gas flow rate = 50:8:2 (sccm)

### (Etching conditions of resist underlayer film with resist intermediate film pattern)

Output: 50 W
Pressure: 20 Pa
Time: 2 min
Etching gas
Ar gas flow rate : CF₄ gas flow rate : O₂ gas flow rate = 50:5:5 (sccm)

### (Etching conditions of SiO₂ film with resist underlayer film pattern)

Output: 50 W
Pressure: 20 Pa
Time: 2 min
Etching gas
Ar gas flow rate : C₅F₁₂ gas flow rate : C₂F₆ gas flow rate : O₂ gas flow rate = 50:4:3:1 (sccm)

### [Evaluation]

The pattern cross-section (the shape of the SiO₂ film after etching) in Example 6, obtained as above, was observed by using an electron microscope (S-4800) manufactured by Hitachi Ltd. It was thus confirmed that any Example in which the underlayer film of the present invention was used was good because the shape of the SiO₂ film after etching in multilayer resist processing was rectangular and no defects were observed.

### (Example 7) Purification of BisF-1-CH4

To a four-neck flask (bottom outlet) having an inner volume of 1000 mL was charged 150 g of a solution (10% by mass) in which BisF-1-CH4 used in Example 1 was dissolved in PGMEA, and heated to 80°C with stirring. Then, 37.5 g of an aqueous oxalic acid solution (pH 1.3) was added thereto, stirred for 5 minutes and then left to stand for 30 minutes. Thus, the resultant was separated to an oil phase and an aqueous phase, and the aqueous phase was removed. This operation was repeated once, and thereafter the resulting oil phase was charged with 37.5 g of ultrapure water, stirred for 5 minutes, and thereafter left to stand for 30 minutes to remove the aqueous phase. This operation was repeated three times, and thereafter the pressure in the flask was reduced to 200 hPa or less with heating to 80°C, to thereby distill off the residual water content and PGMEA by concentration. Thereafter, the resultant was diluted with EL-grade PGMEA (reagent produced by Kanto Chemical Co., Inc.) and the concentration was adjusted to 10% by mass, to thereby provide a BisF-1-CH4 solution in PGMEA, in which the metal content was reduced.

### (Comparative Example 4) Purification method by ion exchange resin

After 25 g of an ion exchange resin (Diaion produced by Mitsubishi Chemical Corporation: SMT100-mixed resin) was swollen by cyclohexanone, it was packed in a Teflon (registered trademark) column, and 500 mL of 1,3-dioxolane was fed thereto to thereby perform solvent substitution. Then, 500 g of a solution (10% by mass) in which BisP-1-CH4 obtained in Example 1 was dissolved in 1,3-dioxolane was fed thereto to thereby provide a BisF-1-CH4 solution in dioxolane.

The contents of various metals in each of a 10% by mass BisF-1-CH4 solution in PGMEA before treatment, and the respective solutions obtained in Example 7 and Comparative Example 4 were measured by ICP-MS. The measurement results are shown in Table 2.

[Table 2]

**Table 2**

| | Metal content (ppb) | | | | | |
|---|---|---|---|---|---|---|
| | Na | Mg | K | Fe | Cu | Zn |
| Before treatment BisF-1-CH4 | >99 | 23.2 | >99 | >99 | 2.7 | 13.6 |
| Example 7 | 2.3 | 1.1 | 0.6 | 2.1 | 0.3 | 0.4 |
| Comparative Example 4 | 0.3 | 0.5 | 1 | >99 | 1.2 | 0.4 |

As described above, the present invention is not limited to the above embodiments and Examples, and can be appropriately modified without departing from the gist thereof.

The disclosure of Japanese Patent Application No. 2015-025371 filed on February 12, 2015 is herein incorporated by reference in its entirety.

In addition, all References, Patent Applications and Technical Standards described in the description are herein incorporated by reference, as if such individual References, Patent Literatures and Technical Standards were specifically and individually stated as being incorporated by reference.

The compound and the resin of the present invention have a relatively high carbon concentration, a relatively low oxygen concentration, a relatively high heat resistance and also a relatively high solvent solubility, and can be applied to a wet process. Then, the material for forming an underlayer film for lithography is obtained by using a compound or a resin having a high heat resistance, a relatively high carbon concentration, a relatively low oxygen concentration and also a high solvent solubility, and having a specified structure, and therefore can form an underlayer film whose degradation is suppressed at high-temperature baking and which is also excellent in etching resistance to oxygen plasma etching or the like. Furthermore, it is also excellent in adhesiveness with a resist layer and therefore can form an excellent resist pattern.

Therefore, a material for forming an underlayer film for lithography, including the compound or the resin of the present invention, and a composition for forming an underlayer film, including the material, can be widely and effectively utilized in various applications in which these properties are required. Therefore, the present invention can be widely and effectively utilized for, for example, an electric insulating material; a resist resin; a sealing resin for a semiconductor; an adhesive for a printed wiring board; an electric laminated board mounted on electrical equipment, electronic equipment, industrial equipment and the like; a matrix resin for a prepreg mounted on electrical equipment, electronic equipment, industrial equipment and the like; a material for a build-up laminated board; a resin for fiber-reinforced plastics; a sealing resin for a liquid crystal display panel; a paint; various coating agents; an adhesive; a coating agent for a semiconductor; a resist resin for a semiconductor; and a resin for forming an underlayer film. In particular, the present invention can be particularly effectively utilized in the field of an underlayer film for lithography and an underlayer film for a multilayer resist.

## Claims

1. A compound represented by the following formula (1). 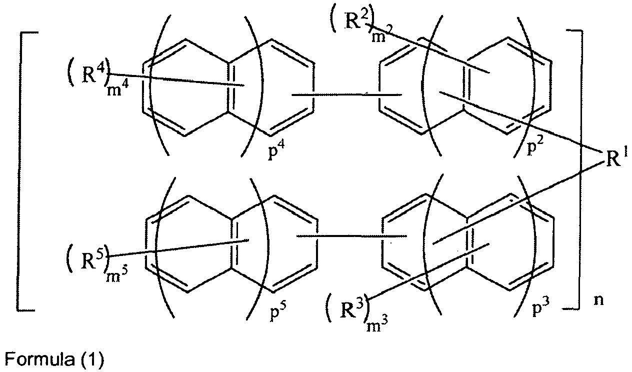 (in formula (1), R¹ represents a 2n-valent group having 1 to 30 carbon atoms, R² to R⁵ each independently represent an alkyl group having 1 to 10 carbon atoms, an aryl group having 6 to 10 carbon atoms, an alkenyl group having 2 to 10 carbon atoms, an alkoxy group having 1 to 30 carbon atoms, a thiol group or a hydroxyl group, provided that at least one R⁴ and/or at least one R⁵ represents an alkoxy group having 1 to 30 carbon atoms, m² and m³ are each independently an integer of 0 to 8, m⁴ and m⁵ are each independently an integer of 0 to 9, provided that m⁴ and m⁵ are not 0 at the same time, n is an integer of 1 to 4, and p² to p⁵ are each independently an integer of 0 to 2.)

2. The compound according to claim 1, wherein at least one R² and/or at least one R³ represents an alkoxy group having 1 to 30 carbon atoms.

3. The compound according to claim 1 or 2, wherein the compound represented by the formula (1) is a compound represented by the following formula (1a). 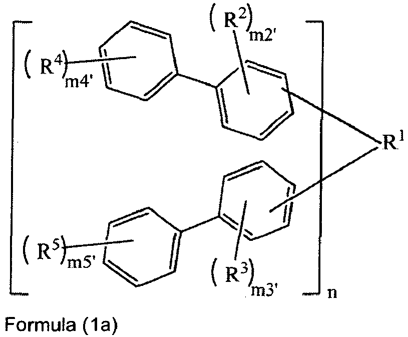 (in formula (1a), R¹ to R⁵ and n are the same as those described in the formula (1), m^{2'} and m^{3'} are each independently an integer of 0 to 4, and m^{4'} and m^{5'} are each independently an integer of 0 to 5, provided that m^{4'} and m^{5'} are not 0 at the same time.)

4. The compound according to claim 3, wherein the compound represented by the formula (1a) is a compound represented by the following formula (1b). 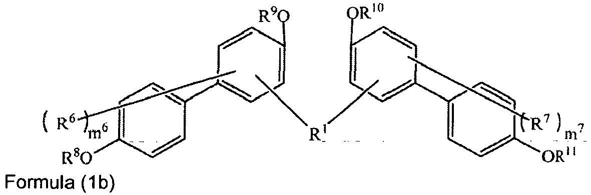 (in formula (1b), R¹ is the same as that described in the formula (1), R⁶ and R⁷ each independently represent an alkyl group having 1 to 10 carbon atoms, an aryl group having 6 to 10 carbon atoms, an alkenyl group having 2 to 10 carbon atoms, a thiol group or a hydroxyl group, R⁸ to R¹¹ each independently represent an alkyl group having 1 to 30 carbon atoms, or a hydrogen atom, provided that at least one of R⁸ to R¹¹ represents an alkyl group having 1 to 30 carbon atoms, and m⁶ and m⁷ are each independently an integer of 0 to 7.)

5. The compound according to claim 4, wherein the compound represented by the formula (1b) is represented by the following formula (BiF-1-CH). 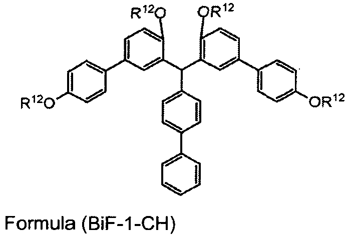 (in formula (BiF-1-CH), each R¹² independently represents a cyclohexyl group or a hydrogen atom, provided that at least one R¹² represents a cyclohexyl group.)

6. A resin obtained with the compound according to any one of claims 1 to 5 as a monomer.

7. The resin according to claim 6, which is obtained by a reaction of the compound according to any one of claims 1 to 5 with a compound having crosslinking reactivity.

8. The resin according to claim 7, wherein the compound having crosslinking reactivity is at least one selected from the group consisting of aldehyde, ketone, carboxylic acid, carboxylic halide, a halogen-containing compound, an amino compound, an imino compound, isocyanate and an unsaturated hydrocarbon group-containing compound.

9. The resin according to claim 6, having a structure represented by the following formula (2). 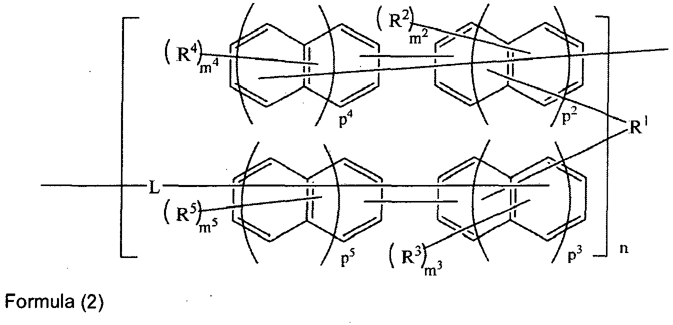 (in formula (2), R¹ represents a 2n-valent group having 1 to 30 carbon atoms, R² to R⁵ each independently represent an alkyl group having 1 to 10 carbon atoms, an aryl group having 6 to 10 carbon atoms, an alkenyl group having 2 to 10 carbon atoms, an alkoxy group having 1 to 30 carbon atoms, a thiol group or a hydroxyl group, provided that at least one R⁴ and/or at least one R⁵ represents an alkoxy group having 1 to 30 carbon atoms, L represents a straight or branched alkylene group having 1 to 20 carbon atoms, or a single bond, m² and m³ are each independently an integer of 0 to 8, m⁴ and m⁵ are each independently an integer of 0 to 9, provided that m⁴ and m⁵ are not 0 at the same time, n is an integer of 1 to 4, and p² to p⁵ are each independently an integer of 0 to 2.)

10. A material for forming an underlayer film for lithography, comprising the compound according to any one of claims 1 to 5 and/or the resin according to any of claims 6 to 9.

11. A composition for forming an underlayer film for lithography, comprising the material for forming an underlayer film for lithography according to claim 10, and a solvent.

12. The composition for forming an underlayer film for lithography according to claim 11, further comprising a crosslinking agent.

13. The composition for forming an underlayer film for lithography according to claim 11 or 12, further comprising an acid generator.

14. An underlayer film for lithography, formed from the composition for forming an underlayer film for lithography according to any one of claims 11 to 13.

15. A resist pattern forming method, comprising forming an underlayer film on a substrate by using the composition for forming an underlayer film according to any one of claims 11 to 13, forming at least one photoresist layer on the underlayer film, thereafter irradiating a required region of the photoresist layer with radiation, and developing it.

16. A circuit pattern forming method, comprising forming an underlayer film on a substrate by using the composition for forming an underlayer film according to any one of claims 11 to 13, forming an intermediate layer film on the underlayer film by using a silicon atom-containing resist intermediate layer film material, forming at least one photoresist layer on the intermediate layer film, thereafter irradiating a required region of the photoresist layer with radiation, developing it to form a resist pattern, thereafter etching the intermediate layer film with the resist pattern as a mask, etching the underlayer film with the obtained intermediate layer film pattern as an etching mask, and etching the substrate with the obtained underlayer film pattern as an etching mask, to form a pattern on the substrate.

17. A purification method of a compound or a resin, comprising a step of bringing a solution including the compound according to any one of claims 1 to 5 or the resin according to any one of claims 6 to 9, and an organic solvent optionally immiscible with water into contact with an acidic aqueous solution for extraction.

18. The purification method according to claim 17, wherein the acidic aqueous solution is an aqueous solution of at least one mineral acid selected from the group consisting of hydrochloric acid, sulfuric acid, nitric acid and phosphoric acid, or an aqueous solution of at least one organic acid selected from the group consisting of acetic acid, propionic acid, oxalic acid, malonic acid, succinic acid, fumaric acid, maleic acid, tartaric acid, citric acid, methanesulfonic acid, phenolsulfonic acid, p-toluenesulfonic acid and trifluoroacetic acid.

19. The purification method according to claim 17 or 18, wherein the organic solvent optionally immiscible with water is toluene, 2-heptanone, cyclohexanone, cyclopentanone, methyl isobutyl ketone, propylene glycol monomethyl ether acetate or ethyl acetate.

20. The purification method according to any one of claims 17 to 19, comprising a step of bringing the solution into contact with the acidic aqueous solution to perform an extraction treatment and thereafter further performing an extraction treatment with water.
